# EUROPÄISCHE PATENTSCHRIFT

(11) **EP 2 121 679 B1**
(45) Veröffentlichungstag und Bekanntmachung des Hinweises auf die Patenterteilung: **15.04.2015**
(21) Anmeldenummer: 08715995.0
(22) Anmeldetag: 22.02.2008
(51) Int. Cl.: C07D 451/06, C07D 451/10

(54) **VERFAHREN ZUR HERSTELLUNG VON AZONIASPIRONORTROPINESTERN**
METHOD FOR PRODUCING AZONIASPIRONORTROPINE ESTERS
PROCÉDÉ DE PRODUCTION D'ESTERS D'AZONIASPIRONORTROPINE

(30) Priorität: 23.02.2007 EP 07102978; 26.02.2007 EP 07103074
(43) Veröffentlichungstag der Anmeldung: 25.11.2009
(73) Patentinhaber: K.H.S. Pharma Holding Gmbh, 55218 Ingelheim (DE)
(72) Erfinder: KREBS, Andreas, 68159 Mannheim (DE); SCHÄFER, Bernd, 76889 Dierbach (DE); KOCHNER, Arno, 67165 Waldsee (DE)
(74) Vertreter: Schnappauf, Georg
(86) Internationale Anmeldenummer: PCT/EP2008/001444
(87) Internationale Veröffentlichungsnummer: WO 2008/101728

(56) Entgegenhaltungen:
- WO-A-2005/080389
- WO-A-2005/101989
- WO-A-2006/134021
- DE-A1- 3 546 218
- MAAG, H. ET AL.: "5-(N-Arylnortropan-3-yl)- and 5-(N-Arylpiperidin-4-yl)-2,4-diaminopyrimi dines. Novel Inhibitors of Dihydrofolate Reductase" HELVETICA CHIMICA ACTA, Bd. 69, 1986, Seiten 887-897, XP002486459
- DE RIDDER, D.J.A. ET AL.: "Effect of Through-Bond Interaction on Conformation and Structure in Rod-Shaped Donor-Acceptor Systems" HELVETICA CHIMICA ACTA, Bd. 86, 2003, Seiten 812-826, XP002486460
- SHEEHAN, J.C. ET AL.: "The Synthesis of Teloidinone and 6-Hydroxytropinone" JOURNAL OF THE AMERICAN CHEMICAL SOCIETY, Bd. 74, 1952, Seiten 3825-3828, XP002486461

## Beschreibung

### TECHNISCHES GEBIET DER ERFINDUNG

Die vorliegende Erfindung betrifft ein verbessertes einstufiges Verfahren zur Herstellung von Azoniaspironortropinestern, insbesondere Trospiumhalogeniden wie beispielsweise Trospiumchlorid, durch Umsetzung von endo-Nortropin oder einem Derivat davon mit einer organischen Dihalogen-Verbindung wie 1,4-Dihalogenbutan und einer α-Hydroxycarbonsäure wie Benzilsäure in Gegenwart einer Base und einem Aktivierungsreagenz ausgewählt aus der Gruppe 1,1'-Carbonyldiimidazol, 1,1'-Thiocarbonyldiimidazol und Thionyldiimidazol. Des Weiteren wird ein verbessertes Verfahren zur Herstellung von geschützten Nortropan-3-on-Verbindungen wie 8-Benzylnortropan-3-on bzw. deren Hydrohalogeniden, insbesondere Hydrochloriden wie N-Benzyltropanon Hydrochlorid, durch Umsetzung eines Amins wie Benzylamin und eines geschützten Dialdehyds mit einer basischen wässrigen Lösung von 1,3-Acetondicarbonsäure beschrieben. Die hergestellten Nortropan-3-on-Verbindungen sind zu endo-Nortropin-Verbindungen umsetzbar, die wiederum in dem erfindungsgemäßen Verfahren zur Herstellung von Azoniaspironortropinestern als Ausgangsstoffe einsetzbar sind.

### STAND DER TECHNIK

Trospiumchlorid ist ein sehr potentes Spasmolytikum. Zur Herstellung dieses Wirkstoffes wurden bisher eine Reihe von Verfahren beschrieben, bei denen Trospiumchlorid ausgehend von der Azoniaspiro-Verbindung der Formel VII hergestellt wird.

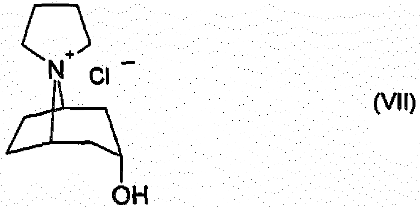

In DE 1194422 und H. Bertholdt, R. Pfleger, W. Schulz, Arzneimittelforschung (1967) 719 wird die Azoniaspiro-Verbindung der Formel VII mit Chlordiphenylessigsäurechlorid umgesetzt. Der Prozess ist umständlich, weil erst durch Substitution des Chlors im Chlordiphenylester der Wirkstoff erhalten wird. Von großem Nachteil ist weiterhin die geringe Löslichkeit der Verbindung VII in nahezu allen aprotischen Lösungsmitteln.

Nach der Lehre der DE 3546218 wird die Verbindung der Formel VII mit Benzilsäure-imidazolid der Formel VIII umgesetzt.

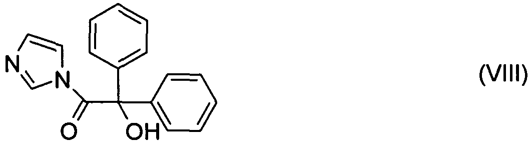

Nachteil des Prozesses ist, dass beide Verbindungen in reiner Form isoliert werden müssen, bevor man sie miteinander zur Reaktion bringen kann. Die Konzentration der Verbindung VII zu Beginn der Reaktion liegt unter 1 Gew.%.

8-Benzylnortropan-3-on ist eine bekannte Vorstufe zur Herstellung von Trospiumchlorid. Zur Herstellung dieses Wirkstoffes wurden bisher eine Reihe von Verfahren beschrieben, die in allen Fällen von den Einsatzstoffen Benzylamin, 2,5-Dimethoxytetrahydrofuran und Acetondicarbonsäure ausgehen, aus denen in einer ersten Stufe im Rahmen einer als Robinson-Schöpf-Reaktion bekannten Umsetzung N-Benzyltropanon Hydrochlorid erhalten wird.

In der WO 2005/080389 ist die Herstellung von 8-Benzylnortropan-3-on durch Zugabe von Acetondicarbonsäure in wässriger Lösung zur salzsauren Lösung eines Überschusses Benzylamin und 2,5-Dimethoxytetrahydrofuran und anschließender Zugabe von Natriumhydrogenphosphat beschrieben. Nach Zugabe des Natriumhydrogenphosphats wird durch Zusatz von 40 %-iger Natronlauge ein pH-Wert von etwa 4,5 eingestellt. Die Reaktion benötigt unter den genannten Bedingungen 2 Tage, um Rohprodukt mit einer Ausbeute von 33 % zu erhalten.

In der WO 2005/101989 ist die schnelle Zugabe von Acetondicarbonsäure als Feststoff und Benzylamin zu einer salzsauren Lösung von 2,5-Dimethoxytetrahydrofuran beschrieben. Weiterer Zusatzstoff ist Natriumacetat. Die Reaktion wird zunächst bei Raumtemperatur gestartet und später bei 50 °C vervollständigt. Dabei wird das Produkt mit einer Ausbeute von 32 % erhalten.

A. Agócs et al. beschreiben in Tetrahedron 2001, 57, 235-239 die Reaktion eines asymmetrisch funktionalisierten Dialdehydes mit Benzylamin und Acetondicarbonsäure. Dabei wird Benzylamin und Acetondicarbonsäure zu dem Dialdehyd in Wasser mit Essigsäure und Natriumacetat in einer Portion gegeben.

G. P. Pollini et al. beschreiben in Synlett 2005, 164-166 die Reaktion von Methylamin mit einem Dialdehyd und Acetondicarbonsäure. Dabei wird der Dialdehyd zu einer wässrigen Lösung von Acetondicarbonsäure und Methylamin gegeben, die durch die Verwendung von Zitronensäure gepuffert ist.

### AUFGABE DER ERFINDUNG

Aufgabe der vorliegenden Erfindung war es, ein verbessertes Verfahren zur Herstellung von Azoniaspironortropinestern wie beispielsweise Trospiumchlorid ausgehend von dem in aprotischen Lösungsmitteln gut löslichen endo-Nortropin oder Derivaten davon bereitzustellen, das die genannten Verbindungen mit einer möglichst geringen Gesamtstufenzahl, in möglichst hoher Gesamtausbeute und in einer für Arzneimittel bzw. Pharmaprodukte akzeptablen Reinheit zugänglich macht.

Weiterhin sollte verbessertes Verfahren zur Herstellung von endo-Nortropin, einem der Ausgangsstoffe der Trospiumchlorid-Synthese, oder Derivaten davon über geschützte Nortropan-3-on-Verbindungen als Zwischenprodukte bereitgestellt werden. Bei der Herstellung von geschützten Nortropan-3-on-Verbindungen entstehen während der Reaktion durch Decarboxylierung der durch die eingesetzte 1,3-Acetondicarbonsäure eingebrachten Carbonsäurefunktionalitäten pro Mol an eingesetzter 1,3-Acetondicarbonsäure zwei Mole Kohlendioxid. Die Freisetzung großer Mengen eines gasförmigen Nebenproduktes stellt für Umsetzungen im technischen Maßstab eine Herausforderung in apparativer bzw. sicherheitstechnischer Hinsicht dar. Dabei ist eine möglichst kontrollierte Gasfreisetzung wünschenswert. Darüber hinaus sollten Bedingungen gefunden werden, unter denen die Herstellung von geschützten Nortropan-3-on-Verbindungen wie 8-Benzylnortropan-3-on Hydrochlorid durch Robinson-Schöpf Reaktion in möglichst hoher Ausbeute und mit einer geringen Menge möglichst leicht abtrennbarer Nebenprodukte bzw. Verunreinigungen durchgeführt werden kann.

### BESCHREIBUNG DER ERFINDUNG SOWIE DER BEVORZUGTEN AUSFÜHRUNGSFORMEN

In einem ersten Aspekt betrifft die Erfindung ein Verfahren zur Herstellung von Azoniaspironortropinester-Halogenid-Verbindungen der Formel I 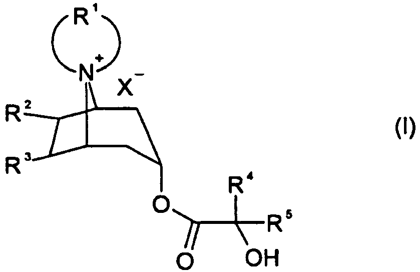 wobei
X ein Halogenatom ist,
R¹ ausgewählt ist aus der Gruppe bestehend aus gegebenenfalls substituierter Alkylengruppe, gegebenenfalls substituierter Alkenylengruppe und der Gruppe -R-Z-R-, wobei jedes R unabhängig voneinander eine kovalente Bindung, eine gegebenenfalls substituierte Alkylengruppe oder eine gegebenenfalls substituierte Alkenylengruppe ist, wobei nicht beide R-Gruppen gleichzeitig eine kovalente Bindung sein können, und Z eine gegebenenfalls substituierte Cycloalkylgruppe, eine gegebenenfalls substituierte Heterocyclylgruppe, eine gegebenenfalls substituierte Arylgruppe oder eine gegebenenfalls substituierte Heteroarylgruppe ist,
R² und R³ unabhängig voneinander ausgewählt sind aus der Gruppe bestehend aus Wasserstoff, gegebenenfalls substituierter Alkylgruppe und gegebenenfalls substituierter Cycloalkylgruppe, und
R⁴ und R⁵ unabhängig voneinander ausgewählt sind aus der Gruppe bestehend aus Wasserstoff, Halogenatom, gegebenenfalls substituierter Alkylgruppe, gegebenenfalls substituierter Alkenylgruppe, gegebenenfalls substituierter Cycloalkylgruppe, gegebenenfalls substituierter Heterocyclylgruppe, gegebenenfalls substituierter Arylgruppe und gegebenenfalls substituierter Heteroarylgruppe,
durch Umsetzung einer Verbindung der Formel II 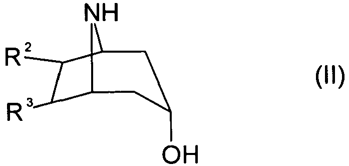 wobei
R² und R³ jeweils die gleiche Bedeutung wie in Formel I besitzen,
mit einer Verbindung der Formel III

X-R¹-X¹ (III)

wobei
X und R¹ jeweils die gleiche Bedeutung wie in Formel I besitzen, und
X¹ ein Halogenatom ist,
und mit einer Verbindung der Formel IV 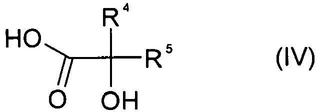 wobei
R⁴ und R⁵ jeweils die gleiche Bedeutung wie in Formel I besitzen,
in Gegenwart einer Base der Formel V 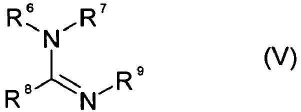 wobei
R⁶, R⁷ und R⁹ gleich oder verschieden sein können und unabhängig voneinander Wasserstoff oder ein gegebenenfalls substituierter, gesättigter oder ungesättigter Kohlenwasserstoffrest sind, wobei R⁷ und R⁹ gemeinsam auch eine gegebenenfalls substituierte, gesättigte oder ungesättigte Kohlenwasserstoffbrücke bilden können, und R⁸ Wasserstoff oder ein gegebenenfalls substituierter, gesättigter oder ungesättigter Kohlenwasserstoffrest oder der Rest -NR¹⁰R¹¹ ist, wobei die Reste R¹⁰ und R¹¹ gleich oder verschieden sein können und Wasserstoff oder ein gegebenenfalls substituierter, gesättigter oder ungesättigter Kohlenwasserstoffrest sind, wobei die Reste R⁶ und R⁸ gemeinsam auch eine gegebenenfalls substituierte Kohlenwasserstoffbrücke bilden können,
und in Gegenwart mindestens eines Aktivierungsreagenzes ausgewählt aus der Gruppe der Aktivierungsreagenzien 1,1'-Carbonyldiimidazol, 1,1'-Thiocarbonyldiimidazol und Thionyldiimidazol.

In einer bevorzugten Ausführungsform betrifft die Erfindung ein Verfahren zur Herstellung von Trospiumhalogenid der Formel la 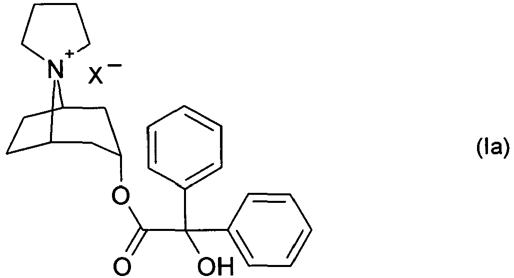 wobei
X Chlor, Brom oder Iod bedeutet,
durch Umsetzung von endo-Nortropin der Formel IIa 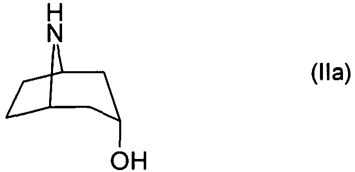 mit 1,4-Dihalogenbutan der Formel IIIa 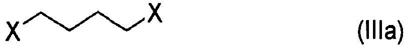 wobei
X die gleiche Bedeutung wie in Formel la besitzt
und mit Benzilsäure der Formel IVa 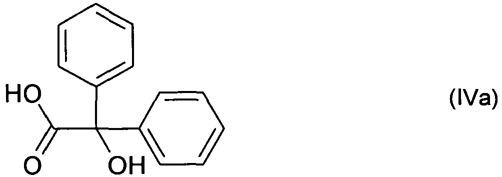 in Gegenwart einer Base der Formel Va 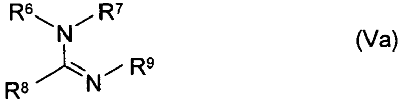 in der die Reste
R⁶, R⁷ und R⁹ gleich oder verschieden sein können und unabhängig voneinander Wasserstoff oder einen gesättigten oder ungesättigten Kohlenwasserstoffrest mit 1 bis 4 Kohlenstoffatomen bedeuten, wobei R⁷ und R⁹ gemeinsam auch eine gesättigte oder ungesättigte Kohlenwasserstoffbrücke mit 3 bis 6 Kohlenstoffatomen bilden können, und
R⁸ Wasserstoff oder einen gesättigten oder ungesättigten Kohlenwasserstoffrest mit 1 bis 4 Kohlenstoffatomen oder den Rest -NR¹⁰R¹¹ bedeutet, wobei die Reste R¹⁰ und R¹¹ gleich oder verschieden sein können und Wasserstoff oder einen gesättigten oder ungesättigten Kohlenwasserstoffrest mit 1 bis 4 Kohlenstoffatomen bedeuten, und wobei die Reste R⁶ und R⁸ gemeinsam auch eine Kohlenwasserstoffbrücke mit 3 bis 6 Kohlenstoffatomen bilden können,
und in Gegenwart mindestens eines Aktivierungsreagenzes ausgewählt aus der Gruppe der Aktivierungsreagenzien 1,1'-Carbonyldiimidazol, 1,1 '-Thiocarbonyldiimidazol und Thionyldiimidazol.

Durch das erfindungsgemäße Verfahren sind Azoniaspironortropinester-Halogenid-Verbindungen wie Trospiumhalogenide der Formel la 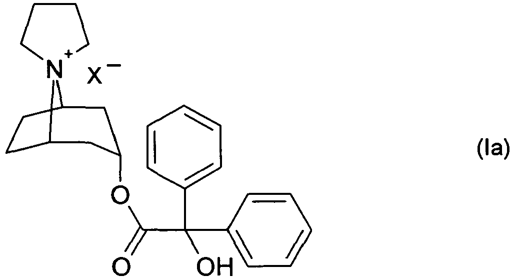 zugänglich, wobei X bevorzugt ein Chlor-, Brom- oder lodatom, mehr bevorzugt ein Chlor- oder Bromatom und besonders bevorzugt ein Chloratom ist. Ein erfindungsgemäß bevorzugtes Verfahrensprodukt ist somit Trospiumchlorid.

Als Ausgangsverbindungen zur Durchführung des erfindungsgemäßen Verfahrens dienen endo-Nortropin-Verbindungen der Formel II, bevorzugt endo-Nortropin (8-Azabicyclo[3.2.1]octan-3-ol), sowie α-Hydroxycarbonsäuren der Formel IV, bevorzugt Benzilsäure (Hydroxydiphenylessigsäure), und organische Dihalogen-Verbindungen der Formel III, bevorzugt 1,4-Dihalogenbutan der Fromel IIIa 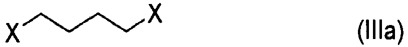 wobei X jeweils die gleiche Bedeutung wie im Verfahrensprodukt der Formel I zukommt und bevorzugt ein Chlor-, Brom- oder lodatom, mehr bevorzugt ein Chlor- oder Bromatom und besonders bevorzugt ein Chloratom ist.

Die erfindungsgemäße Umsetzung wird in Gegenwart einer Base der Formel V 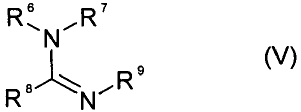
durchgeführt. R⁶, R⁷ und R⁹ können gleich oder verschieden sein und sind bevorzugt unabhängig voneinander Wasserstoff oder ein gesättigter oder ungesättigter Kohlenwasserstoffrest mit 1 bis 4 Kohlenstoffatomen, wobei R⁷ und R⁹ gemeinsam auch eine gesättigte oder ungesättigte Kohlenwasserstoffbrücke mit 3 bis 6 Kohlenstoffatomen bilden können, und
R⁸ ist bevorzugt Wasserstoff oder ein gesättigter oder ungesättigter Kohlenwasserstoffrest mit 1 bis 4 Kohlenstoffatomen oder der Rest -NR¹⁰R¹¹, wobei die Reste R¹⁰ und R¹¹ gleich oder verschieden sein können und Wasserstoff oder ein gesättigter oder ungesättigter Kohlenwasserstoffrest mit 1 bis 4 Kohlenstoffatomen sind, und wobei die Reste R⁶ und R⁸ gemeinsam auch eine Kohlenwasserstoffbrücke mit 3 bis 6 Kohlenstoffatomen bilden können.

Unter den möglichen Amidinen, Guanidinen oder Imidazolen der Formel V sind als erfindungsgemäß bevorzugte Basen zu nennen: Imidazol, 1,8-Diazabicyclo[4.3.0]non-5-en (DBN) und 1,8-Diazabicyclo[5.4.0]undec-7-en (DBU), besonders bevorzugt Imidazol.

Die erfindungsgemäße Umsetzung wird darüber hinaus in Gegenwart, d.h. unter Einsatz eines Aktivierungsreagenzes ausgewählt aus der Gruppe der Aktivierungsreagenzien 1,1'-Carbonyldiimidazol, 1,1'-Thiocarbonyldiimidazol und Thionyldiimidazol durchgeführt. Dabei werden die Aktivierungsreagenzien 1,1'-Carbonyldiimidazol und 1,1'-Thiocarbonyldiimidazol durch Formel VI 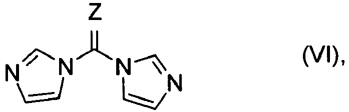 wobei im Fall von 1,1'-Carbonyldiimidazol Z Sauerstoff und im Fall von 1,1'-Thiocarbonyldiimidazol Z Schwefel bedeutet, beschrieben. Thionyldiimidazol weist die Formel IX auf.

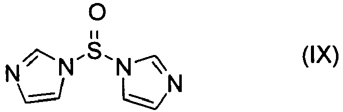

Im Rahmen einer bevorzugten Ausführungsform führt man das erfindungsgemäße Verfahren in Gegenwart von 1,1'-Carbonyldiimidazol (CDI) oder 1,1'-Thiocarbonyldiimidazol, besonders bevorzugt in Gegenwart von 1,1'-Carbonyldiimidazol durch.

In bevorzugten Ausführungsformen der Erfindung sind X und X¹ unabhängig voneinander ein Chlor-, Brom- oder lodatom, bevorzugt ein Chlor- oder Bromatom und besonders bevorzugt ein Chloratom. In einer Ausführungsform sind X und X¹ das gleiche Halogenatom. Der Ring 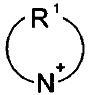 in der Verbindung der Formel I weist vorzugsweise mindestens 4, mehr bevorzugt mindestens 5 Ringatome und vorzugsweise bis zu 10, mehr bevorzugt bis zu 8, mehr bevorzugt bis zu 6 Ringatome auf. R¹ ist vorzugsweise eine gegebenenfalls substituierte Alkylengruppe oder eine gegebenenfalls substituierte Alkenylengruppe, mehr bevorzugt eine Alkylengruppe mit 3 bis 9 Kohlenstoffatomen, vorzugsweise mit 3 bis 7 Kohlenstoffatomen und mehr bevorzugt mit 4 oder 5 Kohlenstoffatomen. Vorzugsweise ist R¹ eine 1,4-Butylengruppe. R² und R³ sind unabhängig voneinander vorzugsweise Wasserstoff oder eine gegebenenfalls substituierte Alkylgruppe, mehr bevorzugt Wasserstoff oder eine Alkylgruppe mit 1 bis 4 Kohlenstoffatomen und besonders bevorzugt Wasserstoff. R⁴ und R⁵ sind unabhängig voneinander vorzugsweise ausgewählt aus der Gruppe bestehend aus gegebenenfalls substituierter Alkylgruppe, gegebenenfalls substituierter Alkenylgruppe, gegebenenfalls substituierter Cycloalkylgruppe und gegebenenfalls substituierter Arylgruppe. Mehr bevorzugt sind R⁴ und R⁵ gegebenenfalls substituierte Arylgruppen und besonders bevorzugt Phenylgruppen.

Die genannten Ausgangsstoffe und Reagenzien können in handelsüblichen Qualitäten und Reinheiten von etwa 90 bis 100 Gew.-% eingesetzt werden und bedürfen vor Einsatz in der Regel keiner speziellen Aufreinigung. Thionyldiimidazol stellt man nach dem Fachmann bekannten Methoden z. B. aus Thionylchlorid und Imidazol her (Takahashi, Shimizu, Ogata, Heterocycles (1985) 1483).

Die erfindungsgemäße Umsetzung wird vorteilhaft in einem organischen Lösungsmittel oder einem Gemisch verschiedener organischer Lösungsmittel durchgeführt. Besonders geeignet sind aprotisch polare organische Lösungsmittel wie beispielsweise N,N-Dimethylformamid (DMF), Dimethylacetamid und N-Methylpyrrolidon oder Gemische derselben. Die genannten Lösungsmittel können in geringen Mengen Wasser enthalten und müssen nicht in speziell vorgetrockneter Form eingesetzt werden. Gemäß einer bevorzugten Ausführungsform wird die erfindungsgemäße Umsetzung in Dimethylformamid als Lösungsmittel durchgeführt. Die genannten Lösungsmittel, bevorzugt DMF, werden vorzugsweise in einer solchen Menge eingesetzt, dass die resultierenden Reaktionsgemische eine Konzentration an endo-Nortropin-Verbindung der Formel II wie beispielsweise endo-Nortropin von etwa 5 bis etwa 30 Gew.-%, bevorzugt etwa 5 bis etwa 10 Gew.-% (bezogen auf das Reaktionsgemisch) aufweisen.

Das Mengenverhältnis der erfindungsgemäß einzusetzenden Ausgangsstoffe kann in breiten Grenzen variiert werden. Unter Berücksichtigung des wirtschaftlichen Aspektes setzt man die endo-Nortropin-Verbindung der Formel II, z.B. endo-Nortropin, und die α-Hydroxycarbonsäure der Formel IV, z.B. Benzilsäure, in einem molaren Verhältnis von etwa 1:3 bis etwa 3:1, bevorzugt in einem molaren Verhältnis von etwa 1:1 bis etwa 1:2 ein. Auch das molare Verhältnis der eingesetzten endo-Nortropin-Verbindung der Formel II, z.B. endo-Nortropin, zu der organischen Dihalogen-Verbindung der Formel III, z.B. 1,4-Dihalogenbutan, wird vorzugsweise in einem Bereich von etwa 3:1 bis etwa 1:3, bevorzugt von etwa 1:1 bis etwa 1:2 gewählt.

Die Reihenfolge für die Zugabe der genannten Ausgangsstoffe und Reagenzien zu dem Reaktionsgemisch ist in der Regel nicht kritisch. Üblicherweise legt man die endo-Nortropin-Verbindung der Formel II, z.B. endo-Nortropin, mit der organischen Dihalogen-Verbindung der Formel III, z.B. 1,4-Dihalogenbutan, in Gegenwart einer Base vor, erhitzt etwa 1 bis etwa 8 h auf Temperaturen von etwa 60 bis 100 °C und versetzt dann dieses Gemisch bei etwa 10 bis 100 °C mit der α-Hydroxycarbonsäure der Formel IV, z.B. Benzilsäure, und dem gewählten Aktivierungsreagenz der Formel VI (1,1'-Carbonyldiimidazol oder 1,1'-Thiocarbonyldiimidazol) bzw. IX (Thionyldiimidazol).

Alternativ kann man auch ein aus dem Aktivierungsreagenz der Formel VI bzw. IX und der α-Hydroxycarbonsäure der Formel IV, z.B. Benzilsäure, gebildetes Gemisch (d.h. die aktivierte α-Hydroxycarbonsäure) zu den restlichen Komponenten, die man zuvor wie vorstehend beschrieben behandelt hat, zugeben. Alternativ kann man auch das wie vorstehend beschrieben behandelte Reaktionsgemisch aus der endo-Nortropin-Verbindung der Formel II, z.B. endo-Nortropin, der organischen Dihalogen-Verbindung der Formel III, z.B. 1,4-Dihalogenbutan, und der Base der Formel V mit der α-Hydroxycarbonsäure der Formel IV, z.B. Benzilsäure, versetzen und schließlich das Aktivierungsreagenz der Formel VI und/oder IX bei 10 - 100 °C zugeben.

Überraschender Weise ist es weiterhin möglich, die endo-Nortropin-Verbindung der Formel II, z.B. endo-Nortropin, mit der α-Hydroxycarbonsäure der Formel IV, z.B. Benzilsäure, und dem Aktivierungsreagenz der Formel VI bzw. IX bei etwa 10 bis etwa 100 °C umzusetzen, anschließend die organische Dihalogen-Verbindung der Formel III, z.B. 1,4-Dihalogenbutan, und die Base der Formel V zuzugeben und etwa 1 bis etwa 8 h auf Temperaturen von etwa 60 bis etwa 100 °C zu erwärmen.

Im Rahmen einer erfindungsgemäß bevorzugten Ausführungsform führt man das Verfahren in Gegenwart von 1,1'-Carbonyldiimidazol oder 1,1'-Thiocarbonyldiimidazol oder Thionyldiimidazol ohne separaten Zusatz einer Base der Formel V durch. Als Base der Formel V dient in diesen Fällen das durch Reaktion von 1,1'-Carbonyldiimidazol oder 1,1'-Thiocarbonyldiimidazol oder Thionyldiimidazol mit der eingesetzten α-Hydroxycarbonsäure der Formel IV, z.B. Benzilsäure, in situ freigesetzte Imidazol, wodurch sich der separate Zusatz von Imidazol oder einer weiteren Base erübrigt. In diesem Fall setzt man vorzugsweise die endo-Nortropin-Verbindung der Formel II, z.B. endo-Nortropin, mit der α-Hydroxycarbonsäure der Formel IV, z.B. Benzilsäure, und dem gewählten Aktivierungsreagenz der Formel VI bzw. IX bei etwa 10 bis etwa 40 °C um, gibt die organische Dihalogen-Verbindung der Formel III, z.B. 1,4-Dihalogenbutan, hinzu und erwärmt etwa 1 bis etwa 8 h auf Temperaturen von etwa 60 bis etwa 100 °C.

Die genannten Ausgangsstoffe und Reagenzien können auch gleichzeitig in ein geeignetes Reaktionsgefäß, z. B. ein Strömungsreaktor oder eine Kesselkaskade, im Sinn einer kontinuierlichen Fahrweise eingetragen werden. In jedem Fall ist es von Vorteil, für die effektive Durchmischung der genannten Reaktionskomponenten zu sorgen, beispielsweise durch Einsatz eines geeigneten Rührwerkes.

Für eine erfolgreiche Durchführung des erfindungsgemäßen Herstellverfahrens ist die Verwendung eines Katalysators nicht unbedingt erforderlich, kann dadurch jedoch vorteilhaft beeinflusst werden. Insbesondere die Verwendung nucleophiler Katalysatoren hat sich als vorteilhaft erwiesen. Ein diesbezüglich erfindungsgemäß bevorzugter Katalysator ist 4-(N,N-Dimethylamino)pyridin (DMAP). Eine bevorzugte Ausführungsform des erfindungsgemäßen Verfahrens ist demgemäß dadurch gekennzeichnet, dass es in Gegenwart von 4-(N,N-Dimethylamino)pyridin als Katalysator durchgeführt wird.

Die erfindungsgemäße Umsetzung kann unter Normaldruck durchgeführt werden. Gewünschtenfalls kann auch bei einem Druck von bis zu 200 bar gearbeitet werden.

Es kann sich empfehlen, die Umsetzung unter einer inerten Schutzgasatmosphäre durchzuführen, wobei als Schutzgase Stickstoff, Kohlendioxid oder Argon zum Einsatz kommen können.

Die Reaktionszeit richtet sich in der Regel nach der Reaktionstemperatur, bei der die eingesetzten Komponenten, d.h. Ausgangsstoffe und Reagenzien miteinander umgesetzt werden. Die Beendigung der Umsetzung kann mit üblichen Verfahren, beispielsweise Kapillarzonenelektrophorese, Ionenaustauschchromatographie, Dünnschichtchromatographie oder HPLC, festgestellt werden.

Das erfindungsgemäße Verfahren eröffnet einen verfahrenstechnisch einfachen und leistungsfähigen Zugang zu Azoniaspironortropinester-Halogenid-Verbindungen der Formel I wie etwa den Trospiumhalogeniden der Formel la. Als verfahrenstechnisch und ökonomisch besonders vorteilhaft ist dabei der Umstand zu werten, dass die erfindungsgemäßen Verfahrensprodukte durch eine einstufige Umsetzung, d.h. ohne Isolierung oder Aufreinigung von Zwischenstufen erhalten werden können. Dadurch werden aufwendige Aufarbeitungs-, Reinigungs- und Isolierungsmaßnahmen eingespart und eine höhere Ausbeute erzielt. Dementsprechend ist die einstufige Verfahrensführung, bei der keine Zwischenprodukte isoliert werden, eine bevorzugte Ausführungsform des erfindungsgemäßen Verfahrens.

Das erfindungsgemäße Verfahren eröffnet beispielsweise einen Zugang zu wahlweise Trospiumchlorid, -bromid oder-iodid, je nachdem, welches 1,4-Halogenbutan der Formel IIIa eingesetzt wird. Unter Einsatz von 1,4-Dibrombutan oder 1,4-Diiodbutan erhält man dementsprechend Trospiumbromid bzw. Trospiumiodid, die gewünschtenfalls jeweils durch Behandlung mit geeigneten Ionentauschern entsprechend der Lehre von DE 119 44 22 in Trospiumchlorid überführt werden können. Zur Herstellung von Trospiumchlorid stellt die Möglichkeit des Einsatzes von kostengünstigerem und sicherheitstechnisch vorteilhafterem 1,4-Dichlorbutan einen weiteren Vorteil des erfindungsgemäßen Verfahrens dar.

Nach erfolgter Umsetzung kann die erhaltene Azoniaspironortropinester-HalogenidVerbindung wie Trospiumhalogenid, bevorzugt Trospiumchlorid, aus dem Reaktionsgemisch durch an sich übliche Verfahren, beispielsweise durch Extraktion und Kristallisation isoliert bzw. weiter aufgereinigt werden. Dazu geeignete Base/Lösungsmittelpaare sind insbesondere solche, bei denen die korrespondierenden unerwünschten Hydrohalogenide im Lösungsmittel bei der Filtrationstemperatur gelöst bleiben, die Azoniasplronortropinester-Halogenid-Verbindung wie z.B. das Trospiumhalogenid jedoch ausfällt. In einer bevorzugten Ausführungsform des erfindungsgemäßen Verfahrens behandelt man dementsprechend die erhaltene Azoniaspironortropinester-Halogenid-Verbindung der Formel I, z.B. Trospiumhalogenid der Formel Ia, mit einem Lösungsmittel, beispielsweise mit N,N-Dimethylformamid, in welchem die Azoniaspironortropinester-Halogenid-Verbindung wie z.B. das Trospiumhalogenid ausfällt und das Hydrohalogenid der eingesetzten Base der Formel V gelöst bleibt.

Dazu kann es erforderlich sein, das gewählte Lösungsmittel in einer solchen Menge einzusetzen, dass die eingesetzte Base der Formel V, bevorzugt Imidazol oder Diazabicyclo[5.4.0]undec-7-en, nicht in Form ihrer Hydrohalogenide, speziell nicht in Form ihrer Hydrochloride ausfallen sondern gelöst bleiben und so leicht von der Azoniaspironortropinester-Halogenid-Verbindung wie Trospiumhalogenid getrennt werden können. In Abhängigkeit von der Art des zur Aufreinigung gewählten Lösungsmittels und der eingesetzten Menge, d.h. in Abhängigkeit der resultierenden Konzentrationen des Produktes bzw. der abzutrennenden Nebenprodukte kann es von Vorteil sein, die Temperatur so zu wählen, dass das gewünschte Verfahrensprodukt ausfällt und die unerwünschten Nebenprodukte gelöst bleiben. Ein bevorzugtes Beispiel hierfür ist 1,8-Diazabicyclo[5.4.0]undec-7-enhydrochlorid in DMF bei etwa 0 °C.

Beschrieben wird auch ein Verfahren zur Herstellung N-substituierter Nortropan-3-on-Verbindungen. Diese Verbindungen lassen sich in endo-Nortropin-Verbindungen der Formel II umsetzen, die als Ausgangsstoffe in dem erfindungsgemäßen Verfahren zur Herstellung von Azoniaspironortropinester-Halogenid-Verbindungen der Formel I dienen. Zum Beispiel kann durch eine erste Umsetzung die Schutzgruppe des Amins abgespalten werden und durch eine weitere Umsetzung die Ketogruppe in eine Hydroxylgruppe umgewandelt werden. Vorzugsweise erfolgt die Abspaltung der Schutzgruppe, die vorzugsweise eine Benzylgruppe ist, mittels Hydrogenolyse, beispielsweise mit Wasserstoff und einem Pd/C Katalysator in einem sauren wässrigen Lösungsmittel wie einem salzsauren Isopropanol/Wasser-Gemisch. Die Reduktion der Ketogruppe erfolgt vorzugsweise mittels Wasserstoff und einem Raney-Nickel-Katalysator in einem basischen wässrigen Lösungsmittel wie einem Isopropanol/Wasser-Gemlsch mit Natriumhydroxid und anschließender Behandlung mit Toluol. Das erhaltene Produkt wird anschließend vorzugsweise mittels azeotroper Destillation aus einem Gemisch aus einem organischen und einem wässrigen Lösungsmittel wie Toluol/Wasser und Kristallisation als freie Base isoliert.

Beschrieben wird daher ein Verfahren zur Herstellung von Nortropan-3-on-Verbindungen der Formel X 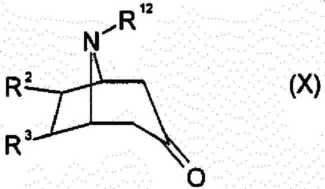 wobei
R² und R³ unabhängig voneinander ausgewählt sind aus der Gruppe bestehend aus Wasserstoff, gegebenenfalls substituierter Alkylgruppe und gegebenenfalls substituierter Cycloalkylgruppe, und
R¹² ausgewählt ist aus der Gruppe bestehend aus gegebenenfalls substituierter Alkylgruppe, Acylgruppe und Carboxylestergruppe,
durch Umsetzung einer Verbindung der Formel XI H

₂N-R¹² (XI)

wobei
R¹² die gleiche Bedeutung wie in Formel X besitzt,
mit einer Verbindung der Formel XII 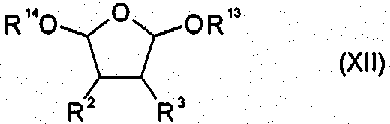 wobei
R² und R³ jeweils die gleiche Bedeutung wie in Formel X besitzen, und
R¹³ und R¹⁴ unabhängig voneinander ausgewählt sind aus der Gruppe bestehend aus Wasserstoff, gegebenenfalls substituierter Alkylgruppe, gegebenenfalls substituierter Alkenylgruppe, gegebenenfalls substituierter Cycloalkylgruppe und gegebenenfalls substituierter Arylgruppe,
und 1,3-Acetondicarbonsäure der Formel XIII 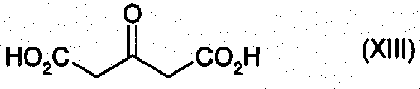 dadurch gekennzeichnet, dass 1,3-Acetondicarbonsäure der Formel XIII in Form einer wässrigen Lösung mit einem pH-Wert von größer als 7 eingesetzt wird.

Beschrieben wird insbesondere ein Verfahren zur Herstellung von 8-Benzylnortropan-3-on (8-Benzyl-8-azablcyclo[3.2.1]octan-3-on) der Formel Xa 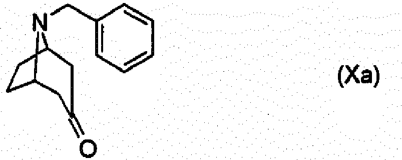 durch Umsetzung von Benzylamin der Formel XIa 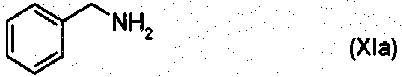 mit einer Verbindung der Formel XIIa 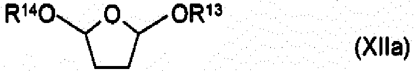 wobei die Reste
R¹³ und R¹⁴ gleich oder verschieden sind und jeweils Wasserstoff oder eine unverzweigte oder verzweigte C₁- bis C₆-Alkylgruppe bedeuten,
und 1,3-Acetondicarbonsäure der Formel XIII 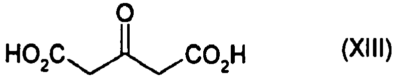 das dadurch gekennzeichnet ist, dass man 1,3-Acetondicarbonsäure der Formel XIII in Form einer wässrigen Lösung mit einem pH-Wert von größer als 7 einsetzt.

Vorzugsweise sind R² und R³ unabhängig voneinander Wasserstoff oder eine gegebenenfalls substituierte Alkylgruppe, mehr bevorzugt Wasserstoff oder eine Alkylgruppe mit 1 bis 4 Kohlenstoffatomen und besonders bevorzugt Wasserstoff. R¹² ist vorzugsweise eine Schutzgruppe, die durch Reduktion oder durch basische oder saure Behandlung, vorzugsweise durch Hydrogenolyse, vom Stickstoff abgespalten werden kann. Beispiele für solche Schutzgruppen sind eine Benzylgruppe, eine tert. Butoxy-carbonylgruppe, eine Benzyloxycarbonylgruppe, eine 9-Fluorenylmethoxycarbonylgruppe und eine Acetylgruppe. Vorzugsweise ist R¹² eine Benzylgruppe. Die Nortropan-3-on-Verbindung der Formel X ist vorzugsweise 8-Benzylnortropan-3-on (8-Benzyl-8-azabicyclo[3.2.1]octan-3-on).

Die 1,3-Acetoncarbonsäure wird in Form einer wässrigen Lösung mit einem pH-Wert von größer als 7, d.h. in vollständig oder teilweise, bevorzugt in vollständig gelöster Form in Wasser oder einem Gemisch aus Wasser und zumindest teilweise mit Wasser mischbaren organischen Lösungsmitteln wie z.B. Methanol oder Ethanol und einer in Wasser löslichen Base eingesetzt.

Hierfür geeignete Basen sind beispielsweise Alkali- oder Erdalkalihydroxide wie zum Beispiel Natriumhydroxid, Lithiumhydroxid, Kaliumhydroxid, Bariumhydroxid oder Calciumhydroxid, bevorzugt Natriumhydroxid, Lithiumhydroxid oder Kaliumhydroxid, besonders bevorzugt Natriumhydroxid. Die genannten Basen können in reiner Form oder in Form von Gemischen untereinander eingesetzt werden. Dabei setzt man die gewählte Base in einer Menge ein, die ausreicht, um die beiden Carbonsäuregruppen der eingesetzten 1,3-Acetondicarbonsäure zu deprotonieren und darüber hinaus den pH-Wert der wässrigen Lösung der 1,3-Acetondicarbonsäure auf einen Wert größer als 7, d.h. auf einen alkalischen pH-Wert zu bringen. Bevorzugt setzt man 1,3-Acetondicarbonsäure in gelöster Form in wässriger Natronlauge, Lithiumhydroxidlösung oder Kalilauge, besonders bevorzugt Natronlauge oder Kalilauge, üblicherweise mit einer Alkalihydroxid-Konzentration von etwa 3 mol/l bis etwa 7 mol/l, bevorzugt etwa 4 mol/l bis etwa 6 mol/l ein. Die resultierenden wässrigen Lösungen von 1,3-Acetondicarbonsäure bzw. dem entsprechenden Dicarboxylat weisen üblicherweise einen pH-Wert von etwa 7 bis 14, bevorzugt etwa 10 bis 14 auf.

Die umzusetzende 1,3-Acetondicarbonsäure liegt in der wässrigen Lösung mit einem pH-Wert von größer als 7 in vollständig deprotonierter Form, d.h. in Form ihres Dicarboxylat-Dianions vor. Die Konzentration der umzusetzenden 1,3-Acetondicarbonsäure bzw. dessen Dianions in den genannten alkalischen Lösungen ist nicht kritisch und kann über einen breiten Bereich variiert werden, beträgt üblicherweise jedoch etwa 1 bis 5, bevorzugt etwa 2 bis 3 mol/l.

Als weitere Ausgangsstoffe zur Durchführung des Verfahrens dienen ein Amin der Formel XI, vorzugsweise Benzylamin, und eine Verbindung der Formel XII, vorzugsweise eine Verbindung der Formel Xlla 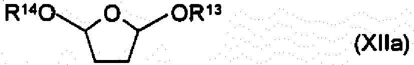 wobei die Reste R¹³ und R¹⁴ gleich oder verschieden, bevorzugt gleich sind und jeweils Wasserstoff oder eine unverzweigte oder verzweigte C₁- bis C₆-Alkylgruppe bedeuten. Unter geradkettiger oder verzweigter C₁- bis C₆-Alkylgruppe sind dabei geradkettige oder verzweigte Alkylreste mit einem bis 6 Kohlenstoffatomen zu verstehen, wie beispielsweise eine Methyl-, Ethyl-, Propyl-, Isopropyl-, Butyl-, Isobutyl-, sek-Butyl-, tert-Butyl-, Pentyl- oder Hexylgruppe, bevorzugt eine Methyl-, Ethyl-, Propyl- oder Isopropy-Igruppe, besonders bevorzugt eine Methyl- oder Ethylgruppe und ganz besonders bevorzugt eine Methylgruppe. Eine erfindungsgemäß besonders bevorzugte Verbindung der Formel XII ist demzufolge 2,5-Dimethoxytetrahydrofuran der Formel XIIb.

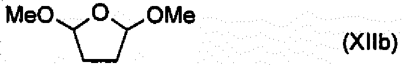

Es können auch Verbindungen der Formel XII, insbesondere Verbindungen der Formel XIIa, eingesetzt werden bei denen einer der Reste R¹³ und R¹⁴ oder beide Reste Wasserstoff bedeuten.

Die genannten Verbindungen der Formeln XI und XII, insbesondere Verbindungen der Formeln XIa und Xlla, können jeweils als solche oder in Form von Lösungen, bevorzugt in Form wässriger Lösungen eingesetzt werden. Als mögliche Lösungsmittel seien auch hierfür Wasser oder Gemische von Wasser und zumindest teilweise mit Wasser mischbare Lösungsmittel wie vorstehend beschrieben genannt.

Vorzugsweise setzt man das Amin der Formel XI, bevorzugt Benzylamin, und die Verbindung der Formel XII, bevorzugt die Verbindung der Formel XIIa, mehr bevorzugt 2,5-Dimethoxytetrahydrofuran, in Form einer wässrigen Lösung mit einem pH-Wert von kleiner als 7 ein. Als Lösungsmittel zur Herstellung derartiger Lösungen können wässrige Lösungen von organischen oder anorganischen Säuren, insbesondere wässrige Salzsäure (Chlorwasserstoffsäure) oder Phosphorsäure oder wässrige Lösungen organischer Carbonsäuren wie beispielsweise Essigsäure verwendet werden.

Vorzugsweise setzt man das Amin der Formel XI, bevorzugt Benzylamin, und die Verbindung der Formel XII, bevorzugt die Verbindung der Formel Xlla, mehr bevorzugt 2,5-Dimethoxytetrahydrofuran, in Form einer Chlorwasserstoffsäure enthaltenden wässrigen Lösung ein. Besonders bevorzugt setzt man die genannten Ausgangsstoffe in Form einer Lösung in Salzsäure ein.

Bei Einsatz des Amins der Formel XI, bevorzugt Benzylamin, und der Verbindung der Formel XII, bevorzugt der Verbindung der Formel XIIa, mehr bevorzugt 2,5-Dimethoxytetrahydrofuran, in Form einer wässrigen Lösung mit einem pH-Wert von kleiner als 7, d.h. in Form einer sauren Lösung, liegt das Amin der Formel XI wie Benzylamin üblicherweise in protonierter Form, beispielsweise in salzsaurer wässriger Lösung in Form seines Hydrochlorids vor.

Die genannten Ausgangsstoffe können entsprechend der zugrunde liegenden Stöchiometrie der Reaktion in etwa äquimolarem Verhältnis zueinander eingesetzt werden, wobei man eine oder zwei Reaktionskomponenten gegebenenfalls auch in leichtem Unterschuss bzw. Überschuss einsetzten kann. Bevorzugt setzt man die Ausgangsstoffe 1,3-Acetondicarbonsäure der Formel XIII zu dem Amin der Formel XI, bevorzugt Benzylamin, und der Verbindung der Formel XII, bevorzugt der Verbindung der Formel XIIa, mehr bevorzugt 2,5-Dimethoxytetrahydrofuran, in einem molaren Verhältnis von etwa 1:1:1 bis etwa 1:0,6:1,2, vorzugsweise bis etwa 1:0,7:1,1 ein.

Zur Durchführung der Umsetzung bringt man die in Form einer wässrigen Lösung mit einem pH-Wert von größer als 7 einzusetzende 1,3-Acetondicarbonsäure in Kontakt mit den beiden weiteren Ausgangsstoffen, dem Amin der Formel XI, bevorzugt Benzylamin, und der Verbindung der Formel XII, bevorzugt der Verbindung der Formel XIIa, mehr bevorzugt 2,5-Dimethoxytetrahydrofuran, wodurch ein Reaktionsgemisch erhalten wird.

Der pH-Wert des dabei erhaltenen Reaktionsgemisches kann generell in einem breiten Rahmen variieren. Die Umsetzung wird üblicherweise unter sauren Bedingungen, d.h. bei einem pH-Wert des Reaktionsgemisches von unter 7, bevorzugt bei einem pH-Wert des Reaktionsgemisches von etwa 1 bis etwa 6, besonders bevorzugt bei einem pH-Wert von etwa 2 bis etwa 5 und insbesondere bevorzugt bei einem pH-Wert von etwa 3 bis etwa 4 durchgeführt. Bei Einsatz von 1,3-Acetondicarbonsäure in Form einer wässrigen Lösung mit einem pH-Wert von größer als 7 gelingt dies beispielsweise durch Einsatz des Amins der Formel XI, bevorzugt Benzylamin, und der Verbindung der Formel XII, bevorzugt der Verbindung der Formel XIIa, mehr bevorzugt 2,5-Dimethoxytetrahydrofuran, in Form einer wässrigen Lösung mit einem pH-Wert von kleiner als 7, d.h. in Form einer sauren Lösung.

Die Umsetzung wird üblicherweise bei Temperaturen von etwa 0°C bis etwa 100 °C, vorzugsweise von etwa 10°C bis 40 °C durchgeführt. Die Umsetzung kann unter Normaldruck durchgeführt werden. Gewünschtenfalls kann auch bei einem Druck von bis zu 200 bar gearbeitet werden.

Die Umsetzung kann durch die geeignete Wahl der Reaktionsbedingungen vorteilhaft beeinflusst werden. Dabei kann es insbesondere vorteilhaft sein, die Reaktionspartner bzw. Ausgangsstoffe in geeigneter Weise miteinander in Kontakt zu bringen bzw. zusammenzuführen. Die Umsetzung kann sowohl kontinuierlich als auch diskontinuierlich durchgeführt werden, d.h. dass die Ausgangsstoffe auf einmal miteinander in Kontakt gebracht bzw. zusammengegeben werden oder allmählich, d.h. über einen längeren Zeitraum verteilt, zusammengeführt werden.

So lässt sich beispielsweise die einzusetzende wässrige Lösung von 1,3-Acetondicarbonsäure in einem geeigneten Reaktionsgefäß, beispielsweise einem Rührkessel, vorlegen und die weiteren Reaktanden, das Amin der Formel XI, bevorzugt Benzylamin, und die Verbindung der Formel XII, bevorzugt die Verbindung der Formel XIIa, mehr bevorzugt 2,5-Dimethoxytetrahydrofuran, in gelöster Form zugeben oder über einen längeren Zeitraum verteilt zudosieren. Alternativ kann man auch das Amin der Formel XI, bevorzugt Benzylamin, und die Verbindung der Formel XII, bevorzugt die Verbindung der Formel XIIa, mehr bevorzugt 2,5-Dimethoxytetrahydrofuran, in fester oder gelöster Form in einem geeigneten Reaktionsgefäß vorlegen und die einzusetzende wässrige Lösung von 1,3-Acetondicarbonsäure mit einem pH-Wert von größer als 7 zugeben bzw. zudosieren. Im Rahmen einer weiteren Ausführungsform des Verfahrens führt man die wässrige Lösung von 1,3-Acetondicarbonsäure mit einem pH-Wert von größer als 7 und die weiteren Reaktionspartner parallel, bevorzugt über einen längeren Zeitraum verteilt, in ein geeignetes Reaktionsgefäß. Dabei ist es in jedem Fall von Vorteil, für gute Durchmischung der Reaktionspartner zu sorgen, beispielsweise durch Einsatz eines Rührwerkes.

Ein bevorzugtes Verfahren ist dadurch gekennzeichnet, dass man eine wässrige Lösung des Amins der Formel XI, bevorzugt Benzylamin, und der Verbindung der Formel XII, bevorzugt der Verbindung der Formel XIIa, mehr bevorzugt 2,5-Dimethoxytetrahydrofuran, und die wässrige Lösung von 1,3-Acetondicarbonsäure mit einem pH-Wert von größer als 7 so miteinander in Kontakt bringt, dass man die wässrige Lösung des Amins der Formel XI, bevorzugt Benzylamin, und der Verbindung der Formel XII, bevorzugt der Verbindung der Formel Xlla, mehr bevorzugt 2,5-Dimethoxytetrahydrofuran, in einem Reaktionsgefäß vorlegt und die wässrige Lösung von 1,3-Acetondicarbonsäure der Formel XIII unter Erhalt eines Reaktionsgemisches zudosiert. Vorzugsweise setzt man dabei eine salzsaure wässrige Lösung des Amins der Formel XI, bevorzugt Benzylamin, und der Verbindung der Formel XII, bevorzugt der Verbindung der Formel XIIa, mehr bevorzugt 2,5-Dimethoxytetrahydrofuran, ein.

Ein weiteres bevorzugtes Verfahren ist dadurch gekennzeichnet, dass man eine wässrige Lösung des Amins der Formel XI, bevorzugt Benzylamin, und der Verbindung der Formel XII, bevorzugt der Verbindung der Formel XIIa, mehr bevorzugt 2,5-Dimethoxytetrahydrofuran, und die wässrige Lösung von 1,3-Acetondicarbonsäure der Formel XIII so miteinander in Kontakt bringt, dass man sie gleichzeitig unter Erhalt eines Reaktionsgemisches in ein Reaktionsgefäß zudosiert. Dabei werden die Ausgangsstoffe über einen längeren Zeitraum verteilt kontinuierlich oder auch portionsweise miteinander in Kontakt gebracht. Von besonderem Vorteil ist es dabei, eine wässrige Lösung des Amins der Formel XI, bevorzugt Benzylamin, und der Verbindung der Formel XII, bevorzugt der Verbindung der Formel XIIa, mehr bevorzugt 2,5-Dimethoxytetrahydrofuran, mit einem pH-Wert von kleiner als 7 einzusetzen. Bevorzugt setzt man eine salzsaure wässrige Lösung der genannten Ausgangsstoffe ein.

Durch geeignete Wahl der Reaktionsbedingungen gelingt es, die im Rahmen der Umsetzung auftretende Freisetzung von größeren Mengen gasförmigen Kohlendioxids in technisch gut handhabbarer Weise zu gestalten. Führt man beispielsweise die umzusetzende 1,3-Acetondicarbonsäure kontinuierlich bzw. portionsweise über einen längeren Zeitrum verteilt in die vorgelegte Gesamtmenge der weiteren Reaktionspartner zu, kann die Freisetzungsgeschwindigkeit des gasförmigen Nebenproduktes kontrolliert werden. Ähnliches trifft für die simultane Zugabe der Ausgangsstoffe zu, falls die Reaktionsbedingungen so gewählt sind, dass die simultan zugegebenen Reaktionspartner zumindest zum größten Teil unmittelbar abreagieren und es somit zu einer kontinuierlichen Freisetzung des gasförmigen Nebenproduktes Kohlendioxid kommt. Dies gelingt insbesondere, falls die Reaktionsbedingungen, insbesondere die pH-Werte der eingesetzten wässrigen Lösungen, so gewählt sind, dass das Reaktionsgemisch, das durch in Kontakt bringen der Ausgangsstoffe erhalten wird, einen pH-Wert von etwa 1 bis etwa 6, besonders bevorzugt von etwa 2 bis etwa 5 und insbesondere bevorzugt von etwa 3 bis etwa 4 aufweist.

Vorteilhafterweise kann man eine Lösung von 1,3-Acetondicarbonsäure in Natronlauge parallel zu dem Amin der Formel XI, bevorzugt Benzylamin, und der Verbindung der Formel XII, bevorzugt die Verbindung der Formel XIIa, mehr bevorzugt 2,5-Dimethoxytetrahydrofuran, in Salzsäure so zudosieren, dass ein pH-Wert von 3,5 konstant während der Reaktion gehalten wird. Sobald die Zugabe der sauren Lösung erfolgt ist, kann der Rest an 1,3-Acetondicarbonsäurelösung zugegeben werden.

Die Reaktionszeit richtet sich üblicherweise nach der Menge der eingesetzten Komponenten und der maximal möglichen Gasabfuhr. Die Beendigung der Umsetzung kann mit üblichen Verfahren, beispielsweise mittels Dünnschichtchromatographie oder HPLC festgestellt werden.

Nach erfolgter Umsetzung setzt man der Reaktionslösung vorteilhaft eine basische Lösung, z.B. Natronlauge, Kalilauge, bevorzugt Natronlauge zu, so dass man ein das Verfahrensprodukt der Formel X, vorzugsweise der Formel Xa, enthaltendes Gemisch erhält, das einen pH-Wert im Bereich von etwa 7 bis 14, bevorzugt im Bereich von etwa 9 bis etwa 11 aufweist.

Zur Isolierung kann die erhaltene Nortropan-3-on-Verbindung der Formel X, zum Beispiel 8-Benzylnortropan-3-on, aus dem Reaktionsgemisch durch an sich übliche Verfahren, beispielsweise durch Extraktion, isoliert werden. Zur Extraktion eignen sich aromatische und gesättigte und ungesättigte aliphatische, verzweigte und unverzweigte Kohlenwasserstoffe mit etwa 5 bis etwa 12 Kohlenstoffatomen, insbesondere Toluol, Pentan, Hexan, Heptan und Essigsäureethylester, ganz besonders Essigsäureethylester. Weiterhin eignen sich aliphatische Ether mit etwa 4 bis etwa 8 Kohlenstoffatomen, beispielsweise Diethylether oder Methyl-tert. Butylether und überkritische Lösungsmittel, wie beispielsweise Kohlendioxid, Propan oder Butan. Die Menge des zur Extraktion zugesetzten Lösungsmittels, bevorzugt des zugesetzten Essigsäureethylesters ist im Allgemeinen nicht kritisch. Üblicherweise beträgt das Volumenverhältnis von organischem Lösungsmittel zu Wasser etwa 0,1:1 bis etwa 1:1, bevorzugt etwa 0,25:1.

Das durch Extraktion in organischer Phase erhaltene Rohprodukt kann im Anschluss durch dem Fachmann an sich bekannte Verfahren getrocknet werden. Vorteilhaft führt man eine azeotrope Trocknung der organischen Phase in Gegenwart eines Alkohols oder Esters als Cosolvens, bevorzugt Methanol, Ethanol, Propanol, Isopropanol, Butanol, insbesondere Essigsäureethylester, durch. Die in Form des freien Amins erhaltene Verbindung der Formel X kann anschließend durch Behandlung mit einer Säure in ihr entsprechendes Säureadditionssalz überführt werden.

Bevorzugt behandelt man die erhaltene Nortropan-3-on-Verbindung der Formel X, zum Beispiel 8-Benzylnortropan-3-on, mit einer Halogenwasserstoffsäure, vorzugsweise Chlorwasserstoffsäure, z.B. in Form einer wässrigen Lösung wie wässriger Salzsäure oder einer alkoholischen Lösung der Halogenwasserstoffsäure, bevorzugt einer isopropanolischen Lösung wie isopropanolischer HCl, und isoliert sie in Form einer Nortropan-3-on-Hydrohalogenid-Verbindung, zum Beispiel in Form von 8-Benzylnortropan-3-on Hydrochlorid der Formel XIVa.

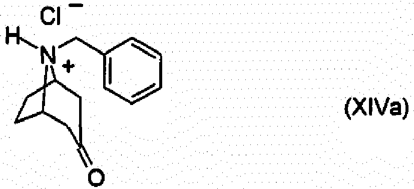

Beschrieben wird demgemäß auch ein Verfahren zur Herstellung von Nortropan-3-on-Hydrohalogenid-Verbindungen der Formel XIV 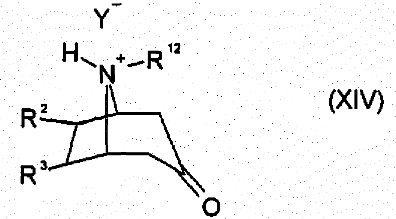 wobei
R² und R³ unabhängig voneinander ausgewählt sind aus der Gruppe bestehend aus Wasserstoff, gegebenenfalls substituierter Alkylgruppe und gegebenenfalls substituierter Cycloalkylgruppe,
R¹² ausgewählt ist aus der Gruppe bestehend aus gegebenenfalls substituierter Alkylgruppe, Acylgruppe und Carboxylestergruppe, und
Y ein Halogenatom ist,
durch Herstellung einer Nortropan-3-on-Verbindung gemäß dem vorstehend beschriebenen Verfahren und anschließender Behandlung der so hergestellten Nortropan-3-on-Verbindung mit einer Halogenwasserstoffsäure.

Beschrieben wird inbesondere ein Verfahren zur Herstellung von 8-Benzylnortropan-3-on Hydrochlorid durch Herstellung von 8-Benzylnortropan-3-on gemäß dem vorstehend beschriebenen Verfahren und anschließender Behandlung das so hergestellten 8-Benzylnortropan-3-ons mit Chlorwasserstoffsäure.

Vorzugsweise sind R² und R³ unabhängig voneinander Wasserstoff oder eine gegebenenfalls substituierte Alkylgruppe, mehr bevorzugt Wasserstoff oder eine Alkylgruppe mit 1 bis 4 Kohlenstoffatomen und besonders bevorzugt Wasserstoff. R¹² ist vorzugsweise eine Schutzgruppe, die durch Reduktion oder durch basische oder saure Behandlung, vorzugsweise durch Hydrogenolyse, vom Stickstoff abgespalten werden kann. Beispiele für solche Schutzgruppen sind eine Benzylgruppe, eine tert. Butoxycarbonylgruppe, eine Benzyloxycarbonylgruppe, eine 9-Fluorenylmethoxycarbonylgruppe und eine Acetylgruppe. Vorzugsweise ist R¹² eine Benzylgruppe. Die Nortropan-3-on-Verbindung der Formel X ist vorzugsweise 8-Benzylnortropan-3-on.

Vorzugsweise ist die Halogenwasserstoffsäure Chlor-, Brom- oder lodwasserstoffsäure, besonders bevorzugt Chlorwasserstoffsäure, und die Nortropan-3-on-Hydrohalogenid-Verbindung der Formel XIV ist entsprechend vorzugsweise eine Nortropan-3-on-Hydrochlorid-, Nortropan-3-on-Hydrobromid- oder Nortropan-3-on-Hydroiodid-Verbindung, besonders bevorzugt eine Nortropan-3-on-Hydrochlorid-Verbindung.

Zur gewünschtenfalls durchzuführenden weiteren Aufreinigung kann der Feststoff der Formel XIV, vorzugsweise der Formel XIVa, in einem Alkohol, beispielsweise in Methanol, suspendiert und gerührt werden. Anschließend kann der Wertstoff durch geeignete Trennverfahren, beispielsweise durch Filtration, von eventuell vorhandenen, im zugesetzten Methanol gelösten Nebenprodukten getrennt werden. Nach einem erneuten Lösungsmitteltausch vorzugsweise durch einen höheren Alkohol, bevorzugt Propanol, Isopropanol oder Butanol, insbesondere bevorzugt Isopropanol, kann die Nortropan-3-on-Verbindung, beispielsweise 8-Benzylnortropan-3-on, in Form seines Hydrohalogenids der Formel XIV, vorzugsweise in Form des Hydrochlorids der Formel XIVa, in sehr guter Ausbeute und Reinheit isoliert werden.

Durch das Verfahren gelingt die Herstellung von Nortropan-3-on-Verbindungen der Formel X, beispielsweise 8-Benzylnortropan-3-on, bzw. deren Säureadditionssalzen wie beispielsweise des Hydrochlorids unter verfahrens- und sicherheitstechnisch vorteilhaften Bedingungen, die sich insbesondere durch eine kontrollierte allmähliche Freisetzung des reaktionsbedingt anfallenden gasförmigen Kohlendioxids auszeichnen. Dies ist insbesondere für Umsetzungen im technischen Maßstab von großer Bedeutung.

Das Verfahrensprodukt fällt in hoher Ausbeute sowie in hoher Reinheit an, wobei sich die gebildeten Nebenprodukte durch einfache Verfahren und ohne Verlust an Wertprodukt leicht abtrennen lassen. Die erhaltene Nortropan-3-on-Verbindungen der Formel X, insbesondere das 8-Benzylnortropan-3-on, und insbesondere das daraus freigesetzte Hydrohalogenid der Formel XIV, vorzugsweise das Hydrochlorid der Formel XIVa, eignen sich damit in besonderem Maße als Ausgangsstoff bzw. Zwischenprodukt zur Herstellung von Pharmawirkstoffen wie Azoniaspironortropinester-Halogenid-Verbindungen der Formel I, insbesondere Trospiumhalogeniden, an die bezüglich Reinheit und Nebenproduktspektrum besonders hohe Anforderungen zu stellen sind.

Der Begriff "Alkylgruppe" betrifft eine einwertige verzweigte oder unverzweigte gesättigte Kohlenwasserstoffkette mit vorzugsweise 1 bis 20 Kohlenstoffatomen, vorzugsweise 1 bis 10 Kohlenstoffatomen, mehr bevorzugt 1, 2, 3, 4, 5 oder 6 Kohlenstoffatomen. Beispiele für diesen Begriff sind Gruppen wie Methyl-, Ethyl-, n-Propyl-, Isopropyl-, n-Butyl-, Isobutyl-, tert.-Butyl-, n-Hexyl-, n-Decyl-, Tetradecylgruppen und dergleichen.

Der Begriff "Alkylengruppe" betrifft einen zweiwertigen Rest einer verzweigten oder unverzweigten gesättigten Kohlenwasserstoffkette mit vorzugsweise 1 bis 20 Kohlenstoffatomen, vorzugsweise 1 bis 10 Kohlenstoffatomen, mehr bevorzugt 1, 2, 3, 4, 5 oder 6 Kohlenstoffatomen. Beispiele für diesen Begriff sind Gruppen wie Methylengruppe (-CH₂-), Ethylengruppe (-CH₂CH₂-), die Propylenisomere (wie -CH₂CH₂CH₂- und -CH(CH₃)CH₂-) und dergleichen.

Der Begriff "Alkenylgruppe" betrifft einen einwertigen Rest einer verzweigten oder unverzweigten ungesättigten Kohlenwasserstoffgruppe mit vorzugsweise 2 bis 20 Kohlenstoffatomen, mehr bevorzugt 2 bis 10 Kohlenstoffatomen und noch mehr bevorzugt 2, 3, 4, 5 oder 6 Kohlenstoffatomen und mit 1 bis 6, vorzugsweise einer Doppelbindung. Bevorzugte Alkenylgruppen umfassen Ethenyl- oder Vinyl- (-CH=CH₂), 1-Propylen- oder Allyl- (-CH₂CH=CH₂), Isopropylen- (-C(CH₃)=CH₂), Bicyclo[2.2.1]heptengruppen und dergleichen. Für den Fall, dass eine Alkenylgruppe an ein Stickstoffatom gebunden ist, kann sich die Doppelbindung nicht in Alpha-Position zu dem Stickstoffatom befinden.

Der Begriff "Alkenylengruppe" betrifft einen zweiwertigen Rest einer verzweigten oder unverzweigten ungesättigten Kohlenwasserstoffgruppe mit vorzugsweise 2 bis 20 Kohlenstoffatomen, mehr bevorzugt 2 bis 10 Kohlenstoffatomen und noch mehr bevorzugt 2, 3, 4, 5 oder 6 Kohlenstoffatomen und mit 1 bis 6, vorzugsweise einer Doppelbindung.

Der Begriff "Cycloalkylgruppe" betrifft eine cyclische Alkylgruppe mit 3 bis 20 Kohlenstoffatomen und einem einzelnen cyclischen Ring oder mehreren kondensierten Ringen. Solche Cycloalkylgruppen umfassen beispielsweise Einzelringstrukturen wie Cyclopropyl-, Cyclobutyl-, Cyclopentyl-, Cyclohexyl-, Cycloheptyl-, Cyclooctylgruppen und dergleichen oder Mehrfachringstrukturen wie Adamantanyl- und Bicyclo[2.2.1]heptangruppen oder eine cyclische Alkylgruppe, an die eine Arylgruppe kondensiert ist, wie eine Indangruppe und dergleichen.

Der Begriff "Heterocyclylgruppe" betrifft eine gesättigte oder teilweise ungesättigte Gruppe mit einem einzelnen Ring oder mehreren kondensierten Ringen, die 1 bis 40 Kohlenstoffatome und 1 bis 10 Heteroatome, vorzugsweise 1 bis 4 Heteroatome, die aus Stickstoff-, Schwefel-, Phosphor- und/oder Sauerstoffatomen ausgewählt sind, in dem Ring aufweist.

Der Begriff "Arylgruppe" betrifft eine aromatische carbocyclische Gruppe mit 6 bis 20 Kohlenstoffatomen und einem einzelnen Ring (wie eine Phenylgruppe) oder mehreren Ringen (wie eine Biphenylgruppe) oder mehreren kondensierten (annelierten) Ringen (wie Naphthyl-, Anthryl-, Tetrahydronaphthyl-, Indangruppen und dergleichen). Bevorzugte Arylgruppen umfassen Phenyl- und Naphthylgruppen.

Der Begriff "Heteroarylgruppe" betrifft eine aromatische Gruppe (d.h. eine ungesättigte Gruppe), die 1 bis 15 Kohlenstoffatome und 1 bis 4 Heteroatome, die ausgewählt sind aus Sauerstoff-, Stickstoff- und Schwefelatom, innerhalb von mindestens einem Ring umfasst.

Der Begriff "Acylgruppe" bezeichnet die Gruppe -C(O)R, worin R ausgewählt ist aus der Gruppe bestehend aus Wasserstoffatom, gegebenenfalls substituierter Alkylgruppe, gegebenenfalls substituierter Cycloalkylgruppe, gegebenenfalls substituierter Heterocyclylgruppe, gegebenenfalls substituierter Arylgruppe und gegebenenfalls substituierter Heteroarylgruppe.

Der Begriff "Carboxylestergruppe" betrifft die Gruppe -C(O)OR, worin R ausgewählt ist aus der Gruppe bestehend aus gegebenenfalls substituierter Alkylgruppe, gegebenenfalls substituierter Cycloalkylgruppe, gegebenenfalls substituierter Heterocyclylgruppe, gegebenenfalls substituierter Arylgruppe und gegebenenfalls substituierter Heteroarylgruppe.

Der Begriff "Halogenatom" oder "Halogen" betrifft Fluor-, Chlor-, Brom- und lodatome.

Der Begriff "Kohlenwasserstoffrest" betrifft eine einwertige Gruppe aus Kohlenstoff- und Wasserstoffatomen. Kohlenwasserstoffreste können geradkettig oder verzweigt sein, Ringstrukturen enthalten und gesättigt oder ungesättigt sein. Vorzugsweise weisen Kohlenwasserstoffreste 1 bis 20 Kohlenstoffatome, vorzugsweise 1 bis 10 Kohlenstoffatome, mehr bevorzugt 1, 2, 3, 4, 5 oder 6 Kohlenstoffatome auf. Beispiele für Kohlenwasserstoffreste sind Alkyl-, Alkenyl-, Alkinyl-, Cycloalkyl- und Arylgruppen, und Kombinationen aus einer Kohlenwasserstoffbrücke und einer Alkyl-, Alkenyl-, Alkinyl-, Cycloalkyl- oder Arylgruppe.

Der Begriff "Kohlenwasserstoffbrücke" betrifft eine zweiwertige Gruppe aus Kohlenstoff- und Wasserstoffatomen. Kohlenwasserstoffbrücken können geradkettig oder verzweigt sein, Ringstrukturen enthalten und gesättigt oder ungesättigt sein. Vorzugsweise weisen Kohlenwasserstoffbrücken 1 bis 20 Kohlenstoffatome, vorzugsweise 1 bis 10 Kohlenstoffatome, mehr bevorzugt 1, 2, 3, 4, 5 oder 6 Kohlenstoffatome auf. Beispiele für Kohlenwasserstoffbrücken sind Alkylen-, Alkenylen-, Alkinylen-, zweiwertige Cycloalkyl- und zweiwertige Arylgruppen und Kombinationen davon.

"Gegebenenfalls" bedeutet, dass das darauf folgend beschriebene Ereignis oder der darauf folgend beschriebene Umstand auftreten oder nicht auftreten kann und dass die Beschreibung Fälle umfasst, wo das Ereignis oder der Umstand auftritt, und Fälle umfasst, bei denen dies nicht der Fall ist.

Der Begriff "substituiert" in Zusammenhang mit einer Verbindung, einer Gruppe oder einem Rest bedeutet, dass in der Verbindung, der Gruppe oder dem Rest ein oder mehrere Wasserstoffatome jeweils durch einen Substituenten ersetzt sind. Vorzugsweise weist eine substituierte Verbindung, eine substituierte Gruppe oder ein substituierter Rest 1, 2, 3, 4 oder 5 Substituenten, mehr bevorzugt 1, 2 oder 3 Substituenten auf. Vorzugsweise sind diese Substituenten unabhängig voneinander ausgewählt aus der Gruppe bestehend aus Alkyl-, Alkenyl-, Alkinyl-, Alkoxy-, Cycloalkyl-, Cycloalkenyl-, Acyl-, Acylamino-, Acyloxy-, Amino-, Aminocarbonyl-, Alkoxycarbonylamino-, Azid-, Cyan-, Halogen-, Hydroxy-, Keto-, Thiocarbonyl-, Carboxy-, Carboxyalkyl-, Arylthio-, Heteroarylthio-, Heterocyclylthio-, Thiol-, Alkylthio-, Aryl-, Aryloxy-, Heteroaryl-, Aminosulfonyl-, Aminocarbonylamino-, Heteroaryloxy-, Heterocyclyl-, Heterocyclooxy-, Hydroxyamino-, Alkoxyamino-, Nitro-, -SO-Alkyl-, -SO-Aryl-, -SO-Heteroaryl-, -SO₂-Alkyl-, -SO₂-Aryl- und -SO₂-Heteroarylgruppen. Wenn nicht anders durch die Definition begrenzt, können alle Substituenten gegebenenfalls weiter mit 1, 2 oder 3 Substituenten substituiert sein, die ausgewählt sind aus Alkyl-, Carboxy-, Carboxyalkyl-, Aminocarbonyl-, Hydroxy-, Alkoxy-, Halogen-, CF₃-, Amino-, substituierten Amino-, Cyan- und -S(O)ₙR-Gruppen, worin R eine Alkyl-, Aryl- oder Heteroarylgruppe ist und n den Wert 0, 1 oder 2 aufweist. Vorzugsweise weist eine substituierte Verbindung, eine substituierte Gruppe oder ein substituierter Rest 1, 2 oder 3 Substituenten ausgewählt aus der Gruppe bestehend aus Alkyl-, Alkenyl-, Cycloalkyl- und Arylgruppe auf.

### BEISPIELE

Die folgenden Beispiele dienen der Erläuterung der Erfindung, ohne sie in irgendeiner Weise zu beschränken:

### Beispiel 1:

1,4-Dichlorobutan (52,4 g, 0,4 mol) und DMF (100 ml) wurden in einem Reaktionsgefäß bei 80 °C vorgelegt und eine Lösung von endo-Nortropin (25,5 g, 0,2 mol), und DBU (88,8 g, 0,3 mol) in N,N-Dimethylformamid (DMF, 150 ml) innerhalb von 1 h zugegeben und das Reaktionsgemisch noch 1 h bei 80°C nachgerührt.

1,1'-Carbonyldiimidazol (48,6 g) und DMF (100 ml) wurden in einem zweiten Reaktionsgefäß vorgelegt und eine Lösung von Benzilsäure (69,2 g, 0,3 mol) in DMF (150 ml) bei einer Temperatur von 20 °C innerhalb von 30 min zugegeben. Das Reaktionsgemisch wurde 1 h bei 20°C gerührt.

N,N-Dimethylaminopyridin (2,5 g, 0,02 mol) und die wie vorstehend beschrieben hergestellte Suspension des Benzilsäureimidazolids wurden bei 80°C innerhalb von 5 min zum Reaktionsgemisch aus 1,4-Dichlorbutan und endo-Nortropin gegeben und das resultierende Gemisch 1 h bei 80°C weitergerührt.

Das Reaktionsgemisch wurde auf 0°C abgekühlt, filtriert und der gelbe Filterrückstand zweimal mit je 50 ml DMF und zweimal mit je 100 ml Aceton gewaschen. Der Filterkuchen wurde mit Stickstoff getrocknet und 82,6 g rohes Trospiumchlorid in Form eines farblosen Feststoffes erhalten.

77 g des Rohproduktes wurden in 535 ml n-Propanol und 5 ml einer 5 - 6 molaren Lösung von HCl in Isopropanol bei einer Temperatur von 90°C gelöst. Nach Filtration wurde die Lösung auf 0°C abgekühlt. Der Filterkuchen wurde erneut zweimal mit je 100 ml Aceton gewaschen und bei 50 °C und 30 mbar für 12 h getrocknet. Man erhielt Trospiumchlorid (61,2 g, 78 %) in Form eines farblosen Feststoffes.

### Beispiel 2:

1,4-Dichlorbutan (5,24 g, 40 mmol) und DMF (10 ml) wurden unter Stickstoff auf 80 °C erwärmt und endo-Nortropin (2,55 g, 20 mmol) und DBU (4,57 g, 30 mmol) gelöst in DMF (15 ml) zugetropft. Nach einer Stunde kühlte man auf 20 °C ab, setzte Benzilsäure (6,92 g, 30 mmol) und CDI (4,86 g, 30 mmol) zu, rührte eine Stunde, setzte DMAP (250 mg, 2 mmol) zu und erhitzte eine Stunde auf 80 °C. Beim Abkühlen auf 0 °C kristallisierte das Wertprodukt aus, das abfiltriert, mit DMF (2 x 5 ml) und Aceton (2 x 10 ml) gewaschen und im Stickstoffstrom getrocknet wurde. Trospiumchlorid, roh (7,1 g, 82,2 %ig, 68 %) wurde in Form eines fast farblosen, kristallinen Feststoffs erhalten.

### Beispiel 3:

Herstellung der Lösung 1: 365,3 g 1,3-Acetondicarbonsäure wurden unter Eisbadkühlung in 1050 g 5-molarer Natronlauge gelöst.

Herstellung der Lösung 2: In 1150 ml voll entsalztem Wasser wurden 626,5 g 32%-iger Salzsäure und 187,5 g Benzylamin gelöst. Zu dieser Lösung wurden 363,8 g 2,5-Dimethoxytetrahydrofuran zugegeben. Diese Mischung wurde für eine halbe Stunde bei Raumtemperatur gerührt.

Lösung 2 und Lösung 1 wurden parallel innerhalb von drei Stunden in einen Rührreaktor mit einem Volumen von 4 l gegeben. Dabei wurde Lösung 2 kontinuierlich in den Reaktor eingetragen und Lösung 2 so zudosiert, dass der pH-Wert des entstandenen Reaktionsgemisches über die gesamte Dauer der Zugabe 3,5 +/- 0,1 betrug. Nach drei Stunden Zugabedauer wurde der Rest an Lösung 1 innerhalb von 10 Minuten zugegeben. Anschließend wurde die erhaltene Reaktionssuspension zwei Stunden bei Raumtemperatur gerührt.

Während dieser Zugabe wurde mit einer Zeitverzögerung von etwa 10 Minuten die kontinuierliche Freisetzung des Kohlendioxids beobachtet. Insgesamt wurden im Laufe der Umsetzung etwa 20 Liter Kohlendioxid freigesetzt.

Daraufhin wurde die Lösung durch Zugabe von Natronlauge auf einen pH-Wert von 10 basisch gestellt. Zur Extraktion des Wertproduktes als freies Amin wurden 300 ml gesättigte Kochsalzlösung sowie 500 ml Essigsäureethylester zugesetzt. Nach erfolgter Phasentrennung wurde die wässrige Phase mit 250 ml Essigsäureethylester nachextrahiert. Die vereinigten organischen Phasen wurden mit 1000 ml Essigsäureethylester versetzt, welche anschließend durch azeotrope Destillation unter vermindertem Druck (35°C, 120 mbar) wieder entfernt wurden.

Anschließend wurde die organische Lösung mit 650 ml Isopropanol versetzt und auf 0°C gekühlt. Zur Isolierung des Produktes wurde dann ein saurer pH-Wert durch die Zugabe von 330 g 5 bis 6 molarer isopropananolischer HCl-Lösung eingestellt. Man erhielt 8-Benzylnortropan-3-on Hydrochlorid in Form eines Feststoffes, der durch Filtration isoliert werden konnte. Das erhaltene Produkt wies einen Gehalt an 8-Benzylnortropan-3-on Hydrochlorid von 75 Gew.- auf.

Zur weiteren Aufreinigung wurde der isolierte Feststoff noch lösungsmittelfeucht in 3570 g Methanol suspendiert. Anschließend wurde durch Filtration unter Verwendung eines Filterhilfsmittels der Feststoffanteil entfernt und schließlich ein Lösungsmitteltausch von Methanol zu Isopropanol durchgeführt und das 8-Benzylnortropan-3-on Hydrochlorid als Feststoff isoliert. Man erhielt 302 g 8-Benzylnortropan-3-on Hydrochlorid mit einem Gehalt von 99 Gew.-% entsprechend einer Ausbeute von 69 %.

### Beispiel 4:

In einem Rundkolben mit einem Volumen von 2 l wurden 460 g Wasser, 250 g 32 %-ige Salzsäure, 75,2 g Benzylamin und 145,2 g 2,5-Dimethoxytetrahydrofuran vorgelegt. Diese Mischung wurde für 30 Minuten bei Raumtemperatur gerührt. Daraufhin wurden innerhalb von 45 Minuten 336 ml einer Lösung von 146 g 1,3-Acetondicarbonsäure und 420 g einer 5 molaren Natronlauge zugegeben wobei der pH-Wert auf 1 stieg.

Verteilt über drei Stunden wurden dann 130 ml einer Mischung von 146 g 1,3-Acetondicarbonsäure und 420 g einer 5 molaren Natronlauge zugegeben. Dabei stieg der pH-Wert auf 4,8. Während dieses Anstiegs des pH-Wertes kam es zur Freisetzung des Kohlendioxids. Anschließend wurde diese Mischung für weitere zwei Stunden bei Raumtemperatur gerührt. Dabei wurde ein pH-Wert von 6,1 erhalten. Daraufhin wurde die Lösung durch Zugabe von Natronlauge auf einen pH-Wert von 10 basisch gestellt. Zur Extraktion des Wertproduktes als freies Amin wurden 120 ml gesättigte Kochsalzlösung sowie 200 ml Essigsäureethylester zugesetzt.

Nach erfolgter Phasentrennung wurde die wässrige Phase zweimal mit 100 ml Essigsäureethylester nachextrahiert. Anschließend wurde unter Normaldruck der Essigsäureethylester entfernt und durch Methanol ersetzt. Daraufhin wurde durch Zugabe von 80 g 32 %-iger Salzsäure ein pH-Wert von kleiner 3 eingestellt. Es wurde noch für weiter 20 Minuten bei Raumtemperatur nachgerührt und die Verunreinigung durch Filtration entfernt. Das Filtrat wurde mit weiteren 980 ml Methanol verdünnt und mit 100 g Aktivkohle behandelt. Nach erneuter Filtration wurde das Lösungsmittel Methanol wieder durch Isopropanol ersetzt und der erhaltene Feststoff durch Filtration isoliert.

Schließlich wurde dieser noch zweimal mit Isopropanol gewaschen. Man erhielt 126,2 g 8-Benzylnortropan-3-on Hydrochlorid mit einem Gehalt von 96 Gew.-% entsprechend einer Ausbeute von 72 %.

## Patentansprüche

1. Verfahren zur Herstellung von Azoniaspironortropinester-Halogenid-Verbindungen der Formel I 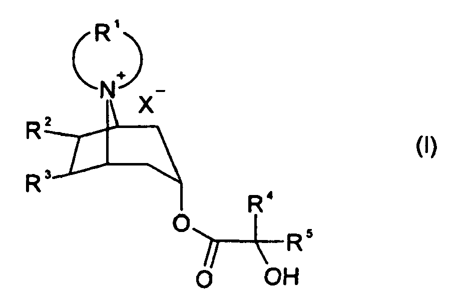 wobei
X ein Halogenatom ist,
R¹ ausgewählt ist aus der Gruppe bestehend aus gegebenenfalls substituierter Alkylengruppe, gegebenenfalls substituierter Alkenylengruppe und der Gruppe -R-Z-R-, wobei jedes R unabhängig voneinander eine kovalente Bindung, eine gegebenenfalls substituierte Alkylengruppe oder eine gegebenenfalls substituierte Alkenylengruppe ist, wobei nicht beide R-Gruppen gleichzeitig eine kovalente Bindung sein können, und Z eine gegebenenfalls substituierte Cycloalkylgruppe, eine gegebenenfalls substituierte Heterocyclylgruppe, eine gegebenenfalls substituierte Arylgruppe oder eine gegebenenfalls substituierte Heteroarylgruppe ist, R² und R³ unabhängig voneinander ausgewählt sind aus der Gruppe bestehend aus Wasserstoff, gegebenenfalls substituierter Alkylgruppe und gegebenenfalls substituierter Cycloalkylgruppe, und
R⁴ und R⁵ unabhängig voneinander ausgewählt sind aus der Gruppe bestehend aus Wasserstoff, Halogenatom, gegebenenfalls substituierter Alkylgruppe, gegebenenfalls substituierter Alkenylgruppe, gegebenenfalls substituierter Cycloalkylgruppe, gegebenenfalls substituierter Heterocyclylgruppe, gegebenenfalls substituierter Arylgruppe und gegebenenfalls substituierter Heteroarylgruppe,
durch Umsetzung einer Verbindung der Formel **II** 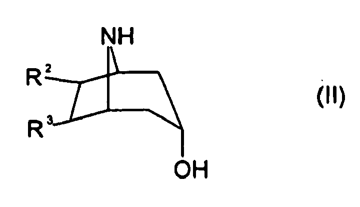 wobei
R² und R³ jeweils die gleiche Bedeutung wie in Formel I besitzen,
mit einer Verbindung der Formel III
X-R¹-X¹ (III)
wobei
X und R¹ jeweils die gleiche Bedeutung wie in Formel I besitzen, und
X¹ ein Halogenatom ist,
und mit einer Verbindung der Formel IV 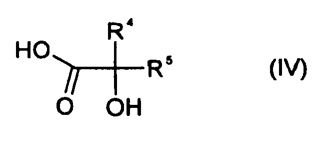 wobei
R⁴ und R⁵ jeweils die gleiche Bedeutung wie in Formel I besitzen, in Gegenwart einer Base der Formel V 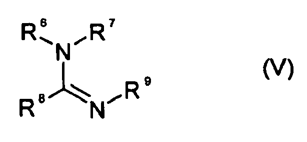 wobei
R⁶, R⁷ und R⁹ gleich oder verschieden sein können und unabhängig voneinander Wasserstoff oder ein gegebenenfalls substituierter, gesättigter oder ungesättigter Kohlenwasserstoffrest sind, wobei R⁷ und R⁹ gemeinsam auch eine gegebenenfalls substituierte, gesättigte oder ungesättigte Kohlenwasserstoffbrücke bilden können,und
R⁸ Wasserstoff oder ein gegebenenfalls substituierter, gesättigter oder ungesättigter Kohlenwasserstoffrest oder der Rest -NR¹⁰R¹¹ ist, wobei die Reste R¹⁰ und R¹¹ gleich oder verschieden sein können und Wasserstoff oder ein gegebenenfalls substituierter, gesättigter oder ungesättigter Kohlenwasserstoffrest sind, wobei die Reste R⁶ und R⁸ gemeinsam auch eine gegebenenfalls substituierte Kohlenwasserstoffbrücke bilden können,
und in Gegenwart mindestens eines Aktivierungsreagenzes ausgewählt aus der Gruppe der Aktivierungsreagenzien 1,1'-Carbonyldiimidazol, 1,1'-Thiocarbonyldiimidazol und Thionyldiimidazol.

2. Verfahren nach Anspruch 1, **dadurch gekennzeichnet, dass** R¹ eine 1,4-Butylengruppe ist.

3. Verfahren nach Anspruch 1 oder 2, **dadurch gekennzeichnet, dass** R² und R³ Wasserstoff sind.

4. Verfahren nach einem der Ansprüche 1 bis 3, **dadurch gekennzeichnet, dass** R⁴ und R⁵ jeweils eine Phenylgruppe sind.

5. Verfahren nach einem der Ansprüche 1 bis 4, **dadurch gekennzeichnet, dass** X und X¹ jeweils ein Chloratom sind.

6. Verfahren nach Anspruch 1 zur Herstellung von Trospiumhalogenid der Formel Ia 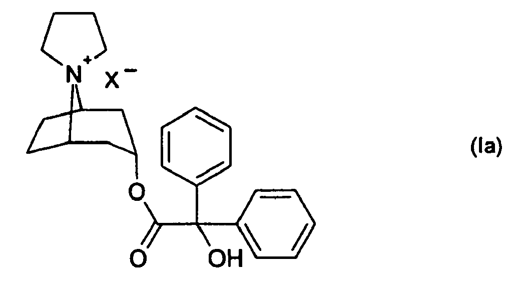 wobei
X Chlor, Brom oder Iod bedeutet,
durch Umsetzung von endo-Nortropin der Formel IIa 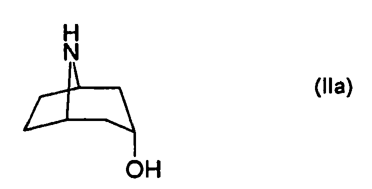 mit 1,4-Dihalogenbutan der Formel IIIa 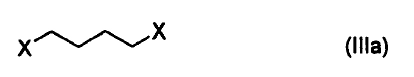 wobei
X jeweils die gleiche Bedeutung wie in Formel la besitzt und mit Benzilsäure der Formel IVa 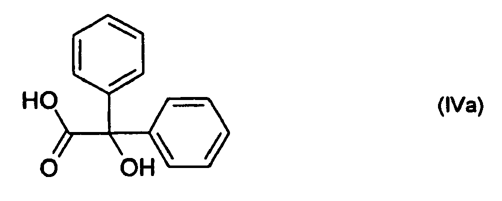 in Gegenwart einer Base der Formel Va 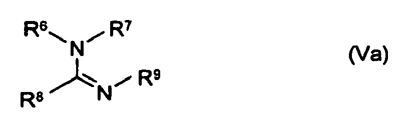 in der die Reste
R6, R⁷ und R⁹ gleich oder verschieden sein können und unabhängig voneinander Wasserstoff oder einen gesättigten oder ungesättigten Kohlenwasserstoffrest mit 1 bis 4 Kohlenstoffatomen bedeuten, wobei R⁷ und R⁹ gemeinsam auch eine gesättigte oder ungesättigte Kohlenwasserstoffbrücke mit 3 bis 6 Kohlenstoffatomen bilden können, und
R⁸ Wasserstoff oder einen gesättigten oder ungesättigten Kohlenwasserstoffrest mit 1 bis 4 Kohlenstoffatomen oder den Rest -NR¹⁰R¹¹ bedeutet, wobei die Reste R¹⁰ und R¹¹ gleich oder verschieden sein können und Wasserstoff oder einen gesättigten oder ungesättigten Kohlenwasserstoffrest mit 1 bis 4 Kohlenstoffatomen bedeuten, und wobei die Reste R⁶ und R⁸ gemeinsam auch eine Kohlenwasserstoffbrücke mit 3 bis 6 Kohlenstoffatomen bilden können,
und in Gegenwart mindestens eines Aktivierungsreagenzes ausgewählt aus der Gruppe der Aktivierungsreagenzien 1,1'-Carbonyldiimidazol, 1,1'-Thiocarbonyldiimidazol und Thionyldiimidazol.

7. Verfahren nach einem der Ansprüche 1 bis 6, **dadurch gekennzeichnet, dass** als Base der Formel V oder Va Imidazol oder 1,8-Diazabicyclo[5.4.0]undec-7-en eingesetzt wird.

8. Verfahren nach einem der Ansprüche 1 bis 7, **dadurch gekennzeichnet, dass** als Base der Formel V oder Va Imidazol eingesetzt wird.

9. Verfahren nach Anspruch 8, **dadurch gekennzeichnet, dass** das eingesetzte Imidazol in situ als Reaktionsprodukt des eingesetzten Aktivierungsreagenzes mit der Verbindung der Formel IV oder IVa gebildet wird.

10. Verfahren nach einem der Ansprüche 1 bis 9, **dadurch gekennzeichnet, dass** die Umsetzung in N,N-Dimethylformamid, N,N-Dimethylacetamid oder N-Methylpyrrolidon oder Gemischen derselben als Lösungsmittel durchgeführt wird.

11. Verfahren nach einem der Ansprüche 1 bis 10, **dadurch gekennzeichnet, dass** die Verbindung der Formel II oder IIa im Reaktionsgemisch in einer Konzentration im Bereich von 5 bis 30 Gew.-% vorliegt.

12. Verfahren nach einem der Ansprüche 1 bis 11, **dadurch gekennzeichnet, dass** das Verfahren in Form eines einstufigen Verfahrens durchgeführt wird und dabei keine Zwischenprodukte isoliert werden.

13. Verfahren nach einem der Ansprüche 1 bis 12, **dadurch gekennzeichnet, dass** die Umsetzung in Gegenwart von 4-(N,N-Dimethylamino)pyridin als Katalysator durchgeführt wird.

14. Verfahren nach einem der Ansprüche 1 bis 13, **dadurch gekennzeichnet, dass** die erhaltene Verbindung der Formel I oder Ia mit einem Lösungsmittel behandelt wird, in dem die Verbindung der Formel I oder la ausfällt und das Hydrohalogenid der eingesetzten Base der Formel V oder Va gelöst bleibt.

15. Verfahren nach Anspruch 14, **dadurch gekennzeichnet, dass** als Lösungsmittel N,N-Dimethylformamid eingesetzt wird.

## Claims

1. A method for producing azoniaspironortropine ester halide compounds of the formula I 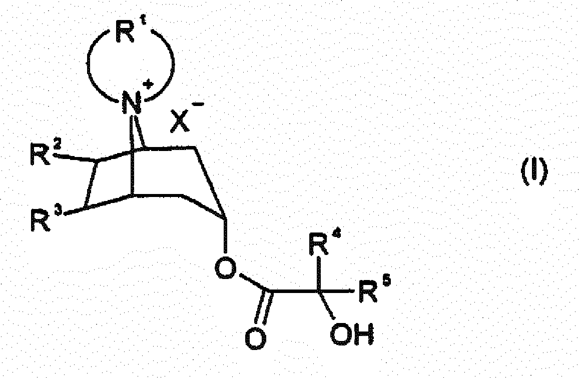 where
X is a halogen atom,
R¹ is selected from the group consisting of optionally substituted alkylene group, optionally substituted alkenylene group and the group -R-Z-R-, where each R, independently of the others, is a covalent bond, an optionally substituted alkylene group or an optionally substituted alkenylene group, where both R groups may not be a covalent bond at the same time, and Z is an optionally substituted cycloalkyl group, an optionally substituted heterocyclyl group, an optionally substituted aryl group or an optionally substituted heteroaryl group, R² and R³, independently of one another, are selected from the group consisting of hydrogen, optionally substituted alkyl group and optionally substituted cycloalkyl group, and
R⁴ and R⁵, independently of one another, are selected from the group consisting of hydrogen, halogen atom, optionally substituted alkyl group, optionally substituted alkenyl group, optionally substituted cycloalkyl group, optionally substituted heterocyclyl group, optionally substituted aryl group and optionally substituted heteroaryl group,
by reacting a compound of the formula II 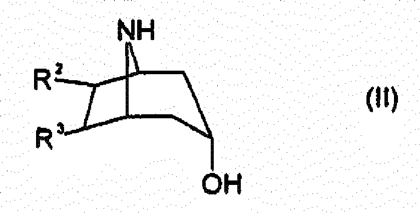 where
R² and R³ each have the same meaning as in formula I,
with a compound of the formula III
X-R¹-X¹ (III)
where
X and R¹ each have the same meaning as in formula I, and
X¹ is a halogen atom,
and with a compound of the formula IV 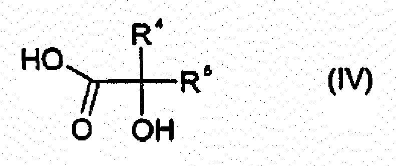 where
R⁴ and R⁵ each have the same meaning as in formula I,
in the presence of a base of the formula V 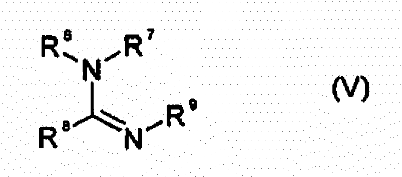 where
R⁶, R⁷ and R⁹ may be identical or different and, independently of one another, are hydrogen or an optionally substituted, saturated or unsaturated hydrocarbon radical, where R⁷ and R⁹ may together also form an optionally substituted, saturated or unsaturated hydrocarbon bridge, and
R⁸ is hydrogen or an optionally substituted, saturated or unsaturated hydrocarbon radical or the radical -NR¹⁰R¹¹, where the radicals R¹⁰ and R¹¹ may be identical or different and are hydrogen or an optionally substituted, saturated or unsaturated hydrocarbon radical, where the radicals R⁶ and R⁸ may together also form an optionally substituted hydrocarbon bridge,
and in the presence of at least one activating reagent selected from the group of activating reagents 1,1'-carbonyldiimidazole, 1,1'-thiocarbonyldiimidazole and thionyldiimidazole.

2. The method as claimed in claim 1, **characterized in that** R¹ is a 1,4-butylene group.

3. The method as claimed in claim 1 or 2, **characterized in that** R² and R³ are hydrogen.

4. The method as claimed in any one of claims 1 to 3, **characterized in that** R⁴ and R⁵ are in each case a phenyl group.

5. The method as claimed in any one of claims 1 to 4, **characterized in that** X and X¹ are in each case a chlorine atom.

6. The method as claimed in claim 1 for producing trospium halide of the formula Ia 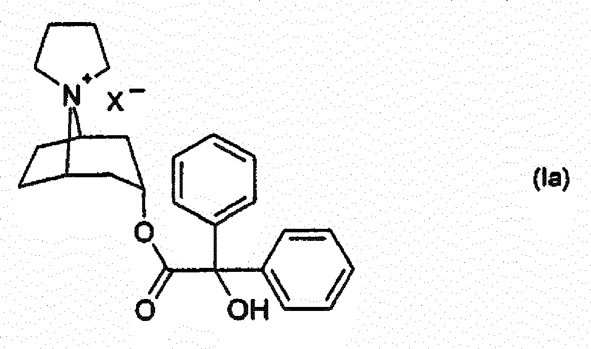 where
X is chlorine, bromine or iodine,
by reacting endo-nortropine of the formula IIa 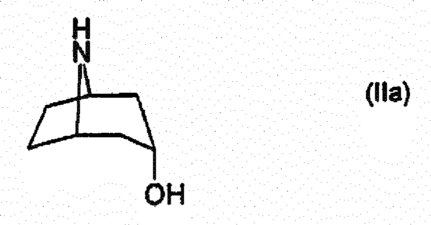 with 1,4-dihalobutane of the formula IIIa 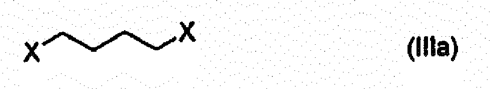 where
X in each case has the same meaning as in formula Ia
and with benzilic acid of the formula IVa 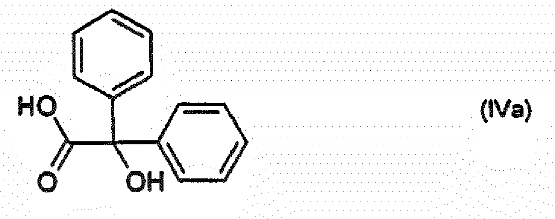 in the presence of a base of the formula Va 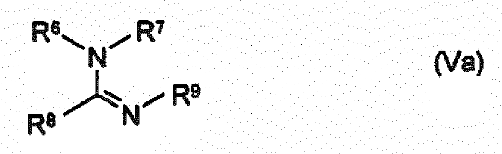 in which the radicals
R⁶, R⁷ and R⁹ may be identical or different and, independently of one another, are hydrogen or a saturated or unsaturated hydrocarbon radical having 1 to 4 carbon atoms, where R⁷ and R⁹ may together also form a saturated or unsaturated hydrocarbon bridge having 3 to 6 carbon atoms, and
R⁸ is hydrogen or a saturated or unsaturated hydrocarbon radical having 1 to 4 carbon atoms or the radical -NR¹⁰R¹¹, where the radicals R¹⁰ and R¹¹ may be identical or different and are hydrogen or a saturated or unsaturated hydrocarbon radical having 1 to 4 carbon atoms, and where the radicals R⁶ and R⁸ may together also form a hydrocarbon bridge having 3 to 6 carbon atoms,
and in the presence of at least one activating reagent selected from the group of activating reagents 1,1'-carbonyldiimidazole, 1,1'-thiocarbonyldiimidazole and thionyldiimidazole.

7. The method as claimed in any one of claims 1 to 6, **characterized in that** imidazole or 1,8-diazabicyclo[5.4.0]undec-7-ene is used as base of the formula V or Va.

8. The method as claimed in any one of claims 1 to 7, **characterized in that** imidazole is used as base of the formula V or Va.

9. The method as claimed in claim 8, **characterized in that** the imidazole used is formed in situ as the reaction product of the activating reagent used with the compound of the formula IV or IVa.

10. The method as claimed in any one of claims 1 to 9, **characterized in that** the reaction is carried out in N,N-dimethylformamide, N,N-dimethylacetamide or N-methylpyrrolidone or mixtures thereof as solvent.

11. The method as claimed in any one of claims 1 to 10, **characterized in that** the compound of the formula II or IIa is present in the reaction mixture in a concentration in the range from 5 to 30% by weight.

12. The method as claimed in any one of claims 1 to 11, **characterized in that** the method is carried out in the form of a single-stage method and no intermediates are isolated during it.

13. The method as claimed in any one of claims 1 to 12, **characterized in that** the reaction is carried out in the presence of 4-(N,N-dimethylamino)pyridine as catalyst.

14. The method as claimed in any one of claims 1 to 13, **characterized in that** the compound of the formula I or Ia obtained is treated with a solvent in which the compound of the formula I or Ia precipitates out and the hydrohalide of the base of the formula V or Va used remains dissolved.

15. The method as claimed in claim 14, **characterized in that** N,N-dimethylformamide is used as solvent.

## Revendications

1. Procédé pour la préparation de composés d'halogénure d'ester
d'azoniaspironortropine de formule I 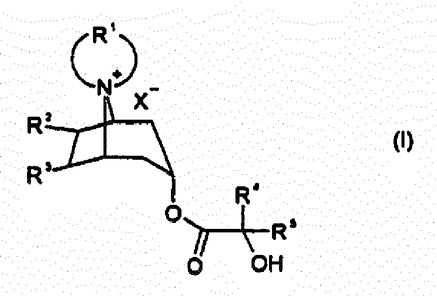 où
X est un atome d'halogène,
R¹ est choisi dans le groupe consistant en groupe alkylène éventuellement substitué, groupe alcénylène éventuellement substitué et le groupe -R-Z-R-, où chaque R est indépendamment l'un de l'autre une liaison covalente, un groupe alkylène éventuellement substitué ou un groupe alcénylène éventuellement substitué, tandis que deux groupes R ne peuvent pas être en même temps une liaison covalente, et Z est un groupe cycloalkyle éventuellement substitué, un groupe hétérocyclyle éventuellement substitué, un groupe aryle éventuellement substitué ou un groupe hétéroaryle éventuellement substitué, R² et R³ sont choisis indépendamment l'un de l'autre dans le groupe consistant en hydrogène, groupe alkyle éventuellement substitué et groupe cycloalkyle éventuellement substitué, et
R⁴ et R⁵ sont choisis indépendamment l'un de l'autre dans le groupe consistant en hydrogène, atome d'halogène, groupe alkyle éventuellement substitué, groupe alcényle éventuellement substitué, groupe cycloalkyle éventuellement substitué, groupe hétérocyclyle éventuellement substitué, groupe aryle éventuellement substitué et groupe hétéroaryle éventuellement substitué,
par réaction d'un composé de formule II 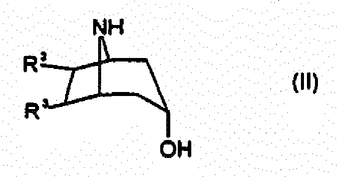 où
R² et R³ ont à chaque fois la même signification que dans la formule I
avec un composé de formule III
X-R¹-X¹ (III)
où
X et R¹ ont la même signification que dans la formule I, et
X¹ est un atome d'halogène,
et avec un composé de formule IV 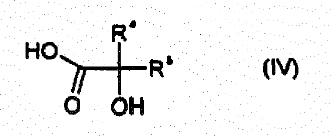 où
R⁴ et R⁵ ont à chaque fois la même signification que dans la formule I,
en présence d'une base de formule V 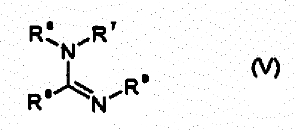 où
R⁶, R⁷ et R⁹ peuvent être identiques ou différents et sont indépendamment l'un de l'autre l'hydrogène ou un reste hydrocarboné saturé ou insaturé, éventuellement substitué, tandis que R⁷ et R⁹ peuvent également former ensemble un pont hydrocarboné saturé ou insaturé, éventuellement substitué, et
R⁸ est l'hydrogène ou un reste hydrocarboné saturé ou insaturé, éventuellement substitué ou le reste -NR¹⁰R¹¹, tandis que les restes R¹⁰ et R¹¹ peuvent être identiques ou différents et sont l'hydrogène ou un reste hydrocarboné saturé ou insaturé, éventuellement substitué, alors que les restes R⁶ et R⁸ peuvent former ensemble également un pont hydrocarboné éventuellement substitué,
et en présence d'au moins un réactif d'activation choisi dans le groupe des réactifs d'activation 1,1'-carbonyldiimidazole, 1,1'-thiocarbonyldiimidazole et thionyldiimidazole.

2. Procédé selon la revendication 1, **caractérisé en ce que** R¹ est un groupe 1,4-butylène.

3. Procédé selon la revendication 1 ou 2, **caractérisé en ce que** R² et R³ sont l'hydrogène.

4. Procédé selon l'une des revendications 1 à 3, **caractérisé en ce que** R⁴ et R⁵ sont à chaque fois un groupe phényle.

5. Procédé selon l'une des revendications 1 à 4, **caractérisé en ce que** X et X¹ sont à chaque fois un atome de chlore.

6. Procédé selon la revendication 1 pour la préparation d'halogénure de trospium de formule Ia 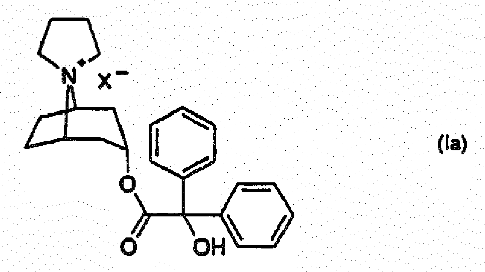 où
X désigne le chlore, le brome ou l'iode,
par réaction d'endo-nortropine de formule IIa 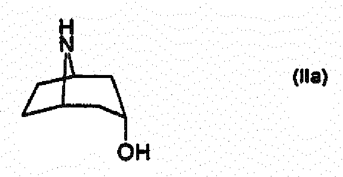 avec le 1,4-dihalogénobutane de formule IIa 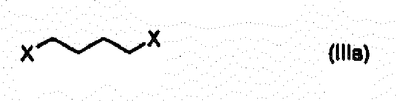 où
X possède à chaque fois la même signification que dans la formule Ia
et avec l'acide benzilique de formule IVa 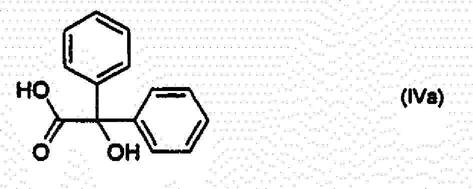 en présence d'une base de formule Va 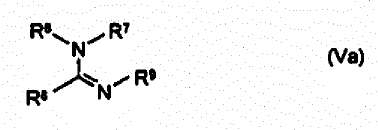 dans laquelle les restes
R⁶, R⁷ et R⁹ peuvent être identiques ou différents et désignent indépendamment l'un de l'autre l'hydrogène ou un reste hydrocarboné saturé ou insaturé ayant 1 à 4 atomes de carbone, tandis que R⁷ et R⁹ peuvent également former ensemble un pont hydrocarboné saturé ou insaturé ayant 3 à 6 atomes de carbone, et
R⁸ désigne l'hydrogène ou un reste hydrocarboné saturé ou insaturé ayant 1 à 4 atomes de carbone ou le reste -NR¹⁰R¹¹, tandis que les restes R¹⁰ et R¹¹ peuvent être identiques ou différents et désignent l'hydrogène ou un reste hydrocarboné saturé ou insaturé ayant 1 à 4 atomes de carbone, et tandis que les restes R⁶ et R⁸ peuvent également former ensemble un pont hydrocarboné ayant 3 à 6 atomes de carbone,
et en présence d'au moins un réactif d'activation choisi dans le groupe des réactifs d'activation 1,1'-carbonyldiimidazole, 1,1'-thiocarbonyldiimidazole et thionyldiimidazole.

7. Procédé selon l'une des revendications 1 à 6, **caractérisé en ce qu'**on utilise comme base de formule V ou Va l'imidazole ou le 1,8-diazabicyclo[5.4.0]undéc-7-ène.

8. Procédé selon l'une des revendications 1 à 7, **caractérisé en ce qu'**on utilise l'imidazole comme base de formule V ou Va.

9. Procédé selon la revendication 8, **caractérisé en ce que** l'imidazole mis en oeuvre est formé in situ en tant que produit de réaction du réactif d'activation introduit avec le composé de formule IV ou IVa.

10. Procédé selon l'une des revendications 1 à 9, **caractérisé en ce que** la réaction est conduite dans le N,N-diméthylformamide, le N,N-diméthylacétamide ou la N-méthylpyrrolidone ou des mélanges de ceux-ci, à titre de solvant.

11. Procédé selon l'une des revendications 1 à 10, **caractérisé en ce que** le composé de formule II ou IIa est présent dans le mélange réactionnel en une concentration dans l'intervalle de 5 à 30 % en poids.

12. Procédé selon l'une des revendications 1 à 11, **caractérisé en ce que** le procédé est mis en oeuvre sous la forme d'un procédé en une étape et on n'isole alors aucun produit intermédiaire.

13. Procédé selon l'une des revendications 1 à 12, **caractérisé en ce que** la réaction est conduite en présence de 4-(N,N-diméthylamino)pyridine en tant que catalyseur.

14. Procédé selon l'une des revendications 1 à 13, **caractérisé en ce que** le composé obtenu de formule I ou Ia est traité avec un solvant, dans lequel le composé de formule I ou Ia précipite et l'halogénhydrate de la base de formule V ou Va introduite reste dissous.

15. Procédé selon la revendication 14, **caractérisé en ce qu'**on utilise comme solvant le N,N-diméthylformamide.
